# EUROPÄISCHE PATENTSCHRIFT

(11) **EP 1 593 339 B1**
(45) Veröffentlichungstag und Bekanntmachung des Hinweises auf die Patenterteilung: **08.09.2010**
(21) Anmeldenummer: 05090128.9
(22) Anmeldetag: 06.05.2005
(51) Int. Cl.: A61B 5/00, G01N 33/543, C12Q 1/00

(54) **Voltammetrischer Mikrofluidiksensor und Verfahren zum direkten oder indirekten Nachweis von glykiertem Hämoglobin**
Voltammetric microfluidic sensor and method for direct or indirect determination of glycated haemoglobin
Capteur microfluidique voltammetrique et procede pour la determination directe ou indirecte de hemoglobine glyquee

(30) Priorität: 06.05.2004 DE 102004023662
(43) Veröffentlichungstag der Anmeldung: 09.11.2005
(73) Patentinhaber: SensLab - Gesellschaft zur Entwicklung und Herstellung bioelektrochemischer Sensoren mbH, 04347 Leipzig (DE)
(72) Erfinder: Gründig, Bernd, Dr., 04318 Leipzig (DE); Hellmich, Robert, Dipl.-Biochem., 04129 Leipzig (DE); Hänsler, Marion, Dr. habil., 04229 Leipzig (DE); Wedig, Heiko, Dipl.-Ing., 04105 Leipzig (DE)
(74) Vertreter: Rasch, Dorit

(56) Entgegenhaltungen:
- EP-A- 1 385 002
- US-A- 5 164 598
- US-A- 5 628 890

## Beschreibung

Die Erfindung betrifft einen voltammetrischen Mikrofluidiksensor und ein Verfahren zum direkten und indirekten Nachweis von glykiertem Hämoglobin. Der Mikrofluidiksensor umfasst ein planares Trägermaterial, das drei geometrisch identisch aufgedruckte und parallel geschaltete voltammetrische Drei-Elektrodenanordnungen aufweist, die jeweils aus Arbeits-, Referenz- und Gegenelektroden bestehen, eine darüber befindliche Isolationsschicht mit Messfenstem über den jeweiligen Elekrodenanordnungen sowie eine mikrostrukturierte Kunststofffolie, welche formschlüssig und irreversibel miteinander verbunden sind. Die Folie weist mindestens eine Probeaufnahmekammer, eine Hämolysekammer sowie mindestens eine Affinitätskammer sowie drei Messkammern auf, welche mit Reagenzien zur Hämolyse von Erythrozyten und gegebenenfalls zur Separation interferierender Blutbestandteile, zur Affinitätsreaktion mit glykiertem Hämoglobin, zur Affinitätsreaktion mit nichtglykiertem Hämoglobin und zum Nachweis von chemischen, elektrochemischen oder enzymatischen Reaktionen dotiert sind. Dieser Mikrofluidiksensor gestattet eine definierte Probeaufnahme, Probeaufsplittung, Probeaufbereitung, affine Reaktion mit glykiertem Hämoglobin, nichtglykiertem Hämoglobin sowie eine serielle, zeitlich versetzte voltammetrische Ausmessung von Gesamthämoglobin, nichtglykiertem Hämoglobin oder glykiertem Hämoglobin und ggf. von Interferenzen und kann mit einem Glucosemeter ausgelesen werden. Bei Diabetes mellitus ist die Bestimmung des HbA_{1c}-Wertes in biologischen Flüssigkeiten, wie z.B. Blut, mittlerweile der wichtigste Parameter zur Langzeitkontrolle und Einstellung des Blutglucosespiegels der Patienten. Die Abkürzung HbA_{1c} steht für Hämoglobin (HbA oder HbA₀), an das sich nichtenzymatisch Kohlenhydratreste gebunden haben. Ende der sechziger Jahre wurde festgestellt, dass speziell Diabetespatienten erhöhte Konzentrationen von derart glykierten Hämoglobinen aufweisen. Durch den Kohlenhydratrest wird die Ladung des nun glykiert vorliegenden Hämoglobins geändert. Es wurden unterschiedliche Hämoglobin-Kohlenhydrat-Addukte identifiziert, die sich durch unterschiedliche Kohlenhydratreste und unterschiedliche Bindungsstellen an das Hämoglobin unterscheiden. Die Fraktion, die mit Abstand in der höchsten Konzentration gefunden wurde und ca. 80 % des glykierten Hämoglobins ausmacht, wird als HbA_{1c} bezeichnet. Bei HbA_{1c} ist ein Glucoserest über die endständige NH₂-Gruppe des Valins in der ß-Untereinheit des Hbs gebunden. Die Glykierung erfolgt nichtenzymatisch und in mehreren Schritten: Glucose reagiert mit der α-Aminogruppe des Valins, aus der eine Schiffsche Base entsteht. Es erfolgt daraus eine sogenannte Amadori-Umlagerung an Glucose, bei der ein stabiler 1-Deoxyfructo-Valin-Komplex entsteht.

Das Ausmaß der Glykierung des Hämoglobins bzw. generell von Serumproteinen ist abhängig sowohl von der Glucose- und Hämoglobinkonzentration im Blut als auch von der Halbwertszeit der Proteine. Demzufolge ist bei Diabetespatienten, die im Durchschnitt erhöhte Glucosekonzentrationen aufweisen, auch der Anteil des glykierten Hämoglobins wesentlich höher als bei Nicht-Diabetikern. Während bei gesunden Personen der Anteil des glykierten Hämoglobins (HbA_{1c}) bei ca. 3 % des Gesamthämoglobins liegt, beträgt der HbA_{1c}-Anteil bei Diabetikern zwischen 5 und 12 % des Gesamthämoglobins. Der Glykierungsgrad wird wesentlich vom Langzeitglucosespiegel im Blut bestimmt, d.h., dass kurzzeitige Glucosespitzenwerte wie sie nach Mahlzeiten auftreten, diesen Parameter nur wenig beeinflussen. Es konnte in einer Reihe von Arbeiten eindrucksvoll bewiesen werden, dass der Anteil des glykierten Hämoglobins sehr gut mit der mittleren Blutglucosekonzentration über die vorangegangenen acht Wochen korreliert. Mittlerweile stellt die HbA_{1c}-Bestimmung den "gold standard" für die Stoffwechselkontrolle bei Diabetikern dar, denn mit der Bestimmung des HbA_{1c}-Wertes erhält der Arzt nicht nur rückwirkend einen Überblick über den durchschnittlichen Glucosespiegel des Patienten, sondern kann den Patienten kontrollierter einstellen und damit auch das Risiko von Folgeschäden der Erkrankung vermindern.

Die Bestimmung von HbA_{1c} erfolgt im Wesentlichen im klinisch-chemischen Labor durch geschultes Fachpersonal. Im Allgemeinen beinhaltet die Bestimmung einen Hämolyseschritt zur Freisetzung des Hämoglobins in der Probe, die Separierung von glykiertem und nicht glykiertem Hämoglobin und schließlich die optische oder elektrochemische Quantifizierung der entsprechenden Hämoglobinfraktion bzw. deren indirekten Nachweis über einen Marker. Von den mittlerweile mehr als 20 bekannten Messmethoden, basieren die am häufigsten angewandten Verfahren auf Prinzipien der Ladungstrennung (Ionenaustauschchromatographie, Elektrophorese) und Affinitätsreaktionen (Affinitätschromatographie, Immunoassays). Bei der Ionenaustauschchromatographie wird die Probe über eine Säule geschickt, die mit einem geeigneten Ionenaustauscher gefüllt ist. Es erfolgt aufgrund der unterschiedlichen Ladungen der einzelnen Hämoglobin-Zuckeraddukte eine Fraktionierung, die im Anschluss eluiert und detektiert werden. Eine dabei bevorzugt genutzte Variante ist die HPLC. Nachteilig ist jedoch die Empfindlichkeit gegenüber kleinsten pH- und Temperaturschwankungen sowie das undifferenzierte Erfassen von labilen Hämoglobinaddukten und carbamyliertem bzw. acetyliertem Hämoglobin. Darüber hinaus verursachen hohe Triglycerid- und Bilirubinkonzentrationen Messwertinterferenzen. Ähnliche Probleme sind von den elektrophöretischen Messverfahren bekannt.

Immunochemische Bestimmungsverfahren nutzen in der Regel die vom Stand der Technik her bekannten Marker und Markierungsverfahren und optische Detektionsverfahren wie sie bei ELISAs, RIAs, Präzipitations- und Agglutinationstests in nasschemischen oder Testreifenformaten Verwendung finden. Im klinischen Alltag haben sich vor allem affinitätsbasierende Indikationsprinzipien bei automatisierten Laborgeräten bzw. ELISAs für die HbA_{1c} - Bestimmung durchgesetzt (wie z.B. DCA 2000, Bayer; Tinaquant II Assay, Roche; Hi-Auto A1c - Analyzer, Kyoto Daiichi Kagaku Co. Ltd, AIA, TOSOH).

Technische Lösungen, bei denen HbA_{1c} elektrochemisch unter Verwendung von Enzymen bestimmt wird, sind ebenfalls bekannt. Durch geeignete Proteasen werden z.B. Fructosylaminosäuren bzw. Ketoaminosäuren von glykiertem Hämoglobin abgespalten und durch Oxidasen umgesetzt. Das resultierende Wasserstoffperoxid kann elektrochemisch, über eine Peroxidase-Farbstoffreaktion oder über die Messung der Sauerstoffabnahme der Oxidasereaktion quantifiziert werden (US 6,127,138 A; EP 1304385 A1; DE 69603472 T2). In US 6,054,039 A ist eine elektrochemische Bestimmung von glykiertem Hämoglobin unter Verwendung eines amperometrischen Sensors zur Bestimmung von Fructosylamin dargestellt, mit dem amperometrisch unter alkalischen Bedingungen in An- und Abwesenheit von lysierten Erythrozyten und mittels eines Redoxmediators Fructosylamin bestimmt und aus der Differenz auf das glykierte Hämoglobin geschlossen wird. Die Verwendung einer Oxygenase oder Oxidase in Kombination mit einem amperometrischen Sensor zur Bestimmung von glykiertem Hämoglobin ist Gegenstand von US 2002/0172992 A1. Das Enzym wird in unmittelbarer Nähe der Kathode als sauerstoffkatalysierende Schicht verwendet. Dabei wird der in der Lösung bekannte (freie) Sauerstoffgehalt gemessen und von dem Gesamtbetrag des Sauerstoffumsatzes abgezogen, der aus freiem Sauerstoff und dem Sauerstoff, der aufgrund der schnellen Gleichgewichtsreaktion aus dem Hämoglobin freigesetzt wird resultiert, wenn sich der freie Sauerstoff in der Lösung durch die Enzymreaktion vermindert. Der freigesetzte Sauerstoff ist je nach Probeaufbereitung proportional zur Gesamthämoglobin-, glykierten Hämoglobin- oder nicht glykierten Hämoglobinkonzentration. Problematisch ist bei dieser Bestimmung die genaue Sauerstoffbestimmung, da der Gelöstsauerstoff auch von physikochemischen Parametern wie Temperatur und Viskosität des Messmediums abhängig ist. Für eine einfache und sichere Kontrolle oder gar zur Selbstkontrolle von Diabetikern, also im Point of Care-Bereich oder Home-Care-Bereich sind diese Systeme demzufolge umgeeignet.

Dafür haben sich insbesondere Teststreifensysteme etabliert, die kommerziell verfügbar sind (z.B. A1cNOW, Metrica; USA Nycard HbA1c, Primus, USA). Beide Testsysteme verwenden laminare Fliessstrecken aus einem porösen Material, in dem über eine Affinitätsreaktion das glykierte Hämoglobin gebunden wird und nach einem Waschschritt der als Label verwendete Farbstoff oder das Hämoglobin reflektometrisch bzw. spektrometrisch quantifiziert wird. Die erforderliche Messzeit beträgt ca. 3 min (Nycard HbA1c) bzw. 8 min (AlcNOW, Metrica). Nachteilig ist jedoch, dass die Systeme in mehreren Schritten gehandhabt werden müssen. Ein Biosensor, der aus einem planaren Sensor oder Teststreifen und einem Kompartimentierten Reaktions- und Messkammeraufsatz besteht ist aus EP 1 385 002 bekannt.

Der Erfindung liegt deshalb die Aufgabe zugrunde, ein System zur HbA_{1c}-Bestimmung in biologischen Flüssigkeiten zu finden und bereitzustellen, das sich vor allem durch eine einfache Handhabbarkeit, schnelle und sichere Messwertverfügbarkeit und geringe erforderliche Probemenge auszeichnet und insbesondere zur Point of Care-Analytik auch für analytische Laien, die ansonsten nur über Erfahrungen mit Glucoseteststreifen zur Selbstkontrolle verfügen (Diabetiker), geeignet ist.

Die Erfindung wird gemäß den Ansprüchen realisiert. Erfindungsgemäß wird die Aufgabe mit einem voltammetrischen Mikrofluidiksensor gelöst, der eine definierte Probeaufnahme, Probeaufsplittung, Probeaufbereitung, affine Reaktion mit glykiertem Hämoglobin und eine serielle, zeitlich versetzte voltammetrische Ausmessung von Hämoglobingehalten einer Probe, nämlich von Gesamthämoglobin, glykiertem Hämoglobin, nichtglykiertem Hämoglobin sowie ggf. von Interferenzen gestattet und in Kombination mit einem angepassten Glucosemeter einen glykierten Hämoglobin-Einschritt-Assay darstellt und zur einfachen und quantitativen Bestimmung von HbA_{1c} geeignet ist.

Der voltammetrische Mikrofluidiksensor umfasst ein planares Trägermaterial 1, eine darüber befindliche Isolationsschicht 1a sowie eine mikrostrukturierte Kunststofffolie 5, welche vorzugsweise gleiche Abmessungen besitzen und formschlüssig und irreversibel miteinander verbunden sind. Das Trägermaterial 1 weist drei geometrisch identisch aufgedruckte und parallel geschaltete voltammetrische Drei-Elektrodenanordnungen auf, die jeweils aus Arbeits-, Referenz- und Gegenelektroden bestehen. Über die Isolationsschicht 1a, die Messfenster über den jeweiligen Elekrodenanordnungen auf dem Träger lässt, ist der Träger 1 mit der Kunststofffolie 5 verbunden. Diese Kunststofffolie umfasst eine Probeaufnahmekammer 6, drei Messkammern 7, 11 und 14, eine Hämolysekammer 9 zur Hämolyse von Erythrozyten und mindestens eine Affinitätskammer 12 zur Bindung von glykiertem Hämoglobin, ggf. eine Rückhaltestrecke 25 zum Rückhalten von nichtglykiertem Hämoglobin, ggf. eine Probeabfallstrecke 27 zur Anreicherung von glykiertem Hämoglobin, sowie Übergangs- und Austrittskanäle. Die Reaktions- und Messkammern sind mit Reagenzien zur Zelllyse, Separation, Affinitätsreaktion und zu chemischen, elektrochemischen oder enzymatischen Reaktionen dotiert. Planarer Träger 1, Isolationsschicht 1a und Folie 5 sind formschlüssig und irreversibel so miteinander verbunden, dass die Probeaufnahmekammer 6 in der Folie 5 mit dem Träger 1 einen Spalt 6a zur Probenaufnahme bildet und die Messkammern jeweils über den Elektrodenanordnungen auf dem Trägermaterial 1 positioniert sind.

Je nach Ziel der Bestimmung von glykiertem Hämoglobin (direkt oder indirekt), weist die Kunststofffolie verschiedene Strukturen auf.

Für den indirekten Nachweis zeigt die Folie 5 die folgenden Mikrostrukturen:
Eine Probeaufnahmekammer 6 ist zum einen mit einer Messkammer 7 (zur Indikation der Probenaufnahme und gegebenenfalls zur Bestimmung von Interferenzen) und zum anderen mit einer Hämolysekammer 9 verbunden. An die Hämolysekammer 9 schließt sich eine zugehörige Messkammer 11 an und gleichzeitig eine Affinitätskammer 12, der ebenfalls eine Messkammer 14 folgt. Des Weiteren umfasst die Folie 5 Übergangs- und Austrittskanäle. Die Reaktionskammern (Probeaufnahmekammer 6, Hämolysekammer 9, Affinitätskammer 12) und die jeweiligen Messkammern 7, 11 und 14) sind mit Reagenzien zur Zelllyse, gegebenenfalls zur Separation von Blutbestandteilen, zur Affinitätsreaktion mit glykiertem Hämoglobin und zum Nachweis chemischer, elektrochemischer oder enzymatischer Reaktionen dotiert. Die Kunstofffolie 5 ist mit dem Träger 1 so verbunden, dass die Probeaufnahmekammer 6 am schmalseitigen Ende einen Spalt 6a zur Probeaufnahme bildet, wobei die Folie 5 so über dem Träger ausgerichtet ist, dass die Messkammern jeweils über den Elektrodenanordnungen, die sich auf dem Trägermaterial 1 befinden, positioniert sind.

Erfindungsgemäß umfasst die Probeaufnahmekammer 6 mindestens ein Antidotmittel zur Reduktion von eventuell vorliegendem Methämoglobin, die Hämolysekammer 9 ist mit mindestens einem hämolytisch wirksamen Detergenz (Hämolyse von Erythrozyten) dotiert und die Affinitätseinrichtung 12 mit mindestens einem affinen Agens zur Bindung von glykiertem Hämoglobin. Die Messkammer 11, die sich an die Hämolysekammer anschließt, weist mindestens einen Redoxmediator zur Ermittlung des Gesamt-hämoglobin auf. Die sich an die Affinitätseinrichtung 12, welche glykiertes Hämoglobin bindet, anschließende Messkammer 14 weist mindestens ein Redoxmediator zur Ermittlung von nicht affinitätsgebundenem Hämoglobin auf.

In einer weiteren Ausführungsvariante des Mikrofluidiksensors weist die mikrostrukturierte Kunststofffolie 5 in der ersten Messkammer 7, die sich (parallel zur Hämolysekammer 9) unmittelbar an die Probeaufnahmekammer 6 anschließt, mindestens einen Redoxmediator auf, der der Ermittlung von Interferenzen durch weitere anwesende elektrochemische Substanzen dient.

In einer weiteren bevorzugten Ausführungsform der Erfindung ist ein erfindungsgemäßer Mikrofluidiksensor zum direkten Nachweis von glykiertem Hämoglobin geeignet, wobei die mikrostrukturierte Kunststofffolie 5 folgende Strukturen aufweist:
Die Probeaufnahmekammer 6 ist mit der Hämolysekammer 9 verbunden welche ein Detergenz zur Hämolyse von Erythrocyten aufweist. Von der Hämolysekammer 9 geht einmal die erste Messkammer 7 ab, die mit einem Redoxmediator zur Ermittlung des GesamtHämoglobingehaltes der Probe dotiert ist, und weiterhin ein sogenannter Fließabschnitt 25 zum Rückhalten von nichtglykiertem Hämoglobin. Dieser Abschnitt ist so dotiert, dass nichtglykiertes Hämoglobin gebunden wird oder aufgrund der Ladung des Dotierungsmaterials in seiner Mobilität stark eingeschränkt wird. Auf diesen Abschnitt 25 folgt ein sich gabelnder Kanal 10, der zum einen zu einer Affinitätskammer 12 führt, die mit mindestens einem affinen Agens zur Bindung von glykiertem Hämoglobin dotiert ist, und an die sich die zweite Messkammer 11 anschließt. Die zweite Messkammer 11 ist dabei mediatordotiert und dient zur Ermittlung von nicht gebundenem restlichen nichtglykierten Hämoglobin. Zum anderen geht von Kanal 10 eine so genannte Verzögerungsstrecke 26 ab, die zur dritten Messkammer 14 führt. Die dritte Messkammer 14 ist sowohl mit einem Agens zur Bindung von glykiertem Hämoglobin als auch mit einem Mediator dotiert und ermittelt zeitverzögert den Gehalt an glykiertem Hämoglobin. Um ein Aufkonzentrieren an glykiertem Hämoglobin in der Messkammer 14 zu ermöglichen, schließt sich an diese ein Probeabfallkanal 27 an.

Die mikrostrukturierte Kunststofffolie 5 besitzt eine Dicke von 100 µm bis 500 µm und wird vorzugsweise mittels einer doppelseitig klebenden Folie 1b, die eine Dicke von 30 µm bis 50 µm hat, mit dem Trägermaterial 1 und der darüber befindlichen Isolationsschicht 1a formschlüssig verbunden. Das Trägermaterial 1 aus handelsüblichen Polymeren weist eine Dicke zwischen 250 µm bis 500 µm auf. Die mittels Heißprägetechnologie erzeugten Mikrostrukturen der Kunststofffolie 5 in Form von Kammern und Kanälen weisen Tiefen zwischen 20 µm und 100 µm auf und sind zwischen 20 µm und 4000 µm breit bzw. lang.

Die Probeaufnahmekammer 6 ist an einer Seite der Folie 5 so angeordnet, dass sie kantenschlüssig mit dem Trägermaterial 1 einen Probeaufnahmespalt 6a bildet. Das Volumen der Probeaufnahmekammer beträgt zwischen 1 µl und 10 µl, vorzugsweise 2 µl bis 5 µl. Das Gesamtvolumen aller anderen Kammern und Verbindungskanäle ist insgesamt nicht größer als das Volumen der Probeaufnahmekammer 6.

Die Hämolyse- und Affinitätskammer 9, 12 und der Fließabschnitt (Rückhaltestrecke) 25 sind derart gefertigt, dass eine sehr große Oberfläche im Vergleich zum durchströmenden Probemedium generiert wird. Deshalb sind die Kammern perlenkettenförmig oder mäanderförmig gestaltet, enthalten in ihrer Kanalstruktur bevorzugt Schikanen oder sind in ihrer Oberfläche mit schachbrettartigen Vertiefungen oder stark porös ausgebildet, so dass ein effizienter Kontakt des Probemediums mit der Oberfläche gesichert ist. Gegebenfalls sind die Einzelsegmente der Affinitätskammer 12 oder der Rückhaltestrecke 25 mit einer porösen Matrix versehen, die ein immobilisiertes Agens zur Bindung oder zur Rückhaltung von glykiertem oder nichtglykiertem Hämoglobin aufweist.

Die Messkammern sind zwischen 200 µm und 500 µm breit und zwischen 1000 µm und 4000 µm lang, so dass das resultierende Messkammervolumen zwischen 0,02 µl und 0,5 µl, vorzugsweise 0,08 bis 0,2 µl beträgt.

Als Material für die Kunststofffolie 5 wird vorzugsweise Polycarbonat oder Polyester verwendet. Die Reagenzien in Probeaufnahme-, Hämolyse-, Affinitäts- und den Messkammern wurden jeweils mittels Siebdruck oder Mikrodispenser aufgetragen.

In der Probeaufnahmekammer 6 ist als Antidotmittel bevorzugt Methylenblau in einer Menge zwischen 0,01 mg und 1 mg, vorzugsweise 0,1 mg deponiert. Methylenblau dient der Reduktion des oxidierten Eisens (Fe³⁺) von Methämoglobin, so dass gegebenenfalls in einer zu analysierenden Blutprobe vorliegendes Methämoglobin zu Hämoglobin reduziert und damit der Gesamthämoglobinwert korrekt erfasst werden kann. NADPH, das aus dem Hexosemonophosphat-Stoffwechselschritt resultiert, wird durch Methylenblau oxidiert und die dabei entstehende Leukoform des Methylenblaus ist der Elektronendonator für die Reduktion des Fe³⁺ zu Fe²⁺. Weitere Antidotmittel können Toluidinblau oder andere chinoide Redoxfarbstoffe sein.

Das in der Hämolysekammer 9 enthaltene hämolytisch wirksame Detergenz ist vorzugsweise Saponin, das in einer Menge zwischen 0,2 mg bis 2 mg aufgetragen ist. Neben Saponin können nichtionogene Tenside wie Ethoxylate des 4-(1,1,3,3-Tetramethylbutyl)phenols (z.B. T. X-100) eingesetzt werden. Die Affinitätskammer 12 in Form einer Kammer oder Säule weist ein gegen glykiertes Hämoglobin affines Agens, vorzugsweise immobilisiert an eine Matrix auf. Als Matrix werden bevorzugt Zellulose, ein Zellulosederivat, Dextran, Sepharosekügelchen, oder Kunststoffe bzw. magnetische Beads mit jeweils funktionalisierten Oberflächen (reaktiven Kopplungsgruppen) verwendet. Das affine Agens ist vorzugsweise ein Boronsäurederivat, Boronsäure oder ein Antikörper. Die Boronatgruppen bilden im Alkalischen mit der cis-Diolstruktur, wie sie in nichtenzymatisch glykierten Proteinen vorliegt, Komplexe aus. Diese Komplexierung ist für das gesamte glykierte Hämoglobin spezifisch, nicht aber für glykierte bzw. glykosylierte Serumproteine (Brownlee et al., Diabetes 29(1980) 1044-1047, Gould et al., Anal. Chim. Biochim., 21(1984), 16-21). Diese Bindung ist relativ unempfindlich gegenüber Schwankungen von Temperatur und pH-Wert. Hämoglobinvarianten oder labile Aldiminformen interferieren nicht mit der HbA_{1c}-Messung.

Die Messkammern, die sich an Hämolyse- und Affinitätskammer anschließen, sind jeweils mit einem Redoxmediator dotiert, der jeweils in einer gleichen Menge zwischen 2 mg und 20 mg pro Messkammer über der gesamten Elektrodenanordnung gleichmäßig verteilt eingebracht wird. Als Mediator wird ein Chinon, Chinonderivat, ein chinoider Redoxfarbstoff aus der Gruppe der Phenazine, Phenoxazine, Phenothiazine, Indamine, Metallocene, Tetrathiavulvalen oder ein redoxaktiver Metallkomplex, vorzugsweise Ferricyanid, verwendet, der das Hämoglobin oxidiert. Der aus der Reduktion des Elektronenmediators an der Arbeitselektrode resultierende Stromfluss ist aufgrund des stöchiometrischen Umsatzes der Hämoglobinkonzentration direkt proportional. Für den Fall, dass auch die erste Messkammer 7, die sich an die Probeaufnahmekammer 6 anschließt, ebenfalls mit einem Mediator dotiert ist, können somit Interferenzen bestimmt werden, da noch keine Hämolyse erfolgt ist.

In einer bevorzugten Ausführungsvariante weist der planare mittels Siebdruck gefertigte Mikrofluidiksensor auf seinem Trägermaterial 1 drei parallel geschaltete und geometrisch identisch aufgedruckte voltammetrische Drei-Elektrodenanordnungen 2, 3, 4 auf und ist mit der Kunststofffolie 5, in die mittels Heißprägetechnologie Mikrostrukturen in Form von Kammern und Kanälen erzeugt wurden, formschlüssig verbunden. Das in der Folie 5 erzeugte Mikrofluidiksystem besteht zum indirekten Nachweis von glykiertem Hämoglobin aus einer Probeaufnahmekammer 6, der ersten Messkammer 7, die von der Probeaufnahmekammer 6 direkt abzweigt, einem Übergangskanal 8 von der Probe aufnahmekammer 6 zu der Hämolysekammer 9, einem weiteren von der Hämolysekammer 9 abgehenden Austrittskanal 10, der sich gabelt und dessen erster Abzweig über einen Kanal 13 in die zur Hämolysekammer gehörende zweite Messkammer 11 und dessen zweiter Abzweig in die Affinitätskammer oder - säule 12 ggf. mit Einzelsegmenten 12a mündet. Die gegenüberliegende Austrittsöffnung der Affinitätskammer 12 ist über einen Verbindungskanal mit der zugehörigen dritten Messkammer 14 verbunden. Die drei Messkammern führen in Auslasskanäle 15, 16, 17 mit Austrittsöffnungen 15a, 16a, 17a.

Jede der drei Messkammern 7, 11, 14, ist über einer voltammetrischen Drei-Elektrodenanordnung aus Arbeits-, Referenz-, und Gegenelektrode 18, 18a, 19, 19a, 20, 20a positioniert. Die drei Kohlenstoff-Elektroden der drei Anordnungen 2, 3, 4 sind jeweils untereinander parallel geschalten und über elektrisch leitende Kohlenstoffleiterzüge 21, 22, 23 untereinander verbunden. Über die Leiterzüge ist die Isolationsschicht 1a gedruckt, die über den Elektrodenflächen jeweils vorzugsweise rechteckige Aussparungen 2a, 3a, 4a aufweist und damit die Messfensterflächen der Elektrodenanordnungen 2, 3, 4 definiert. Die Leiterzüge sind am Ende des Sensorsupports als Kontaktflächenelemente 21a, 22a, 23a ausgebildet, so dass der Sensor elektrisch mit einem geeigneten Glucosemeter kontaktiert werden kann, das die erforderliche Polarisationsspannung bereitstellt und die Signalverarbeitung übernimmt.

In den Messkammern sind jeweils in identischer Weise und mit den gleichen Elektrodengeometrien nacheinander drei Kohlenstoffelektroden angeordnet. Jede der Elektroden ist zwischen 200 µm und 500 µm breit und zwischen 400 µm und 1000 µm lang.

In der bevorzugten Ausführung sind in der ersten sich an die Probeaufnahmekammer anschließenden Messkammer 7 vom Eingang bis zum Ausgang der Probe Referenzelektrode 19a, Gegenelektrode 20a und Arbeitselektrode 18a nacheinander angeordnet und in den beiden anderen Messkammern 11, 14 sind vom Eingang bis zum Ausgang der Probe Referenzelektrode 19, Arbeitselektrode 18 und Gegenelektrode 20 nacheinander angeordnet. Die drei Arbeits-, Referenz- und Gegenelektroden 18, 18a, 19, 19a, 20, 20a sind jeweils kreuzungsfrei miteinander über Kohlenstoffleiterbahnen 21, 22, 23 verbunden und werden über gemeinsame Kontaktflächen 21a, 22a, 23a von einem potentiostatisch betriebenen Glucosemeter mit einer Polarisationsspannung versorgt.

Alternativ enthält zum direkten Nachweis eine bevorzugte Kunsstofffolie 5 die Probeaufnahmekammer (6) mit Antidotmittel. Am Ende der Probenaufnahmekammer befindet sich mittig eine kreisförmige Öffnung 6b, und ein Übergangskanal 8, der zur Hämolysekammer 9 mit hämolytischem Detergenz führt. Am Ende der Hämolysekammer 9 geht ein Kanal 7a ab, der zur ersten mediatorhaltigen Messkammer 7 führt. Die Hämolysekammer geht direkt in einen Fließabschnitt 25, über, dessen Einzelsegmente eine poröse Matrix enthalten, die mit einem affinen Agens dotiert ist. Der darauf folgende sich gabelnden Kanal 10 führt einmal zur Affinitätskammer 12, mit immobilisiertem affinem Agens, der die zweite mediatorhaltige Messkammer 11 folgt. Zum anderen geht der Kanal 10 in eine Verzögerungsstrecke 26, über, die zur dritten Messkammer 14 führt. Die mediatorhaltige Messkammer 14 weist zusätzlich eine poröse Matrix mit immobilisiertem, affinen Agens auf. An die Messkammer 14 schließt sich der Probeabfallkanal 27 an. Darüber hinaus enthält die bevorzugte Folie 5 jeweils an die Messkammern bzw. an den Probeabfallkanal sich anschließende Austrittskanäle 15, 16, 17 mit Austrittsöffnungen 15a ,15b, 15c. Auf dem planaren Trägermaterial 1 sind mittels Siebdruck die Elektrodenflächen 18, 18a, 19, 19a, 20, 20a, 18b, 19b, 20b bzw. Kohlenstoffleiterzüge - und Kontaktflächen 21, 21a, 22, 22a, 23, 23a und die Isolationsschicht 1a aufgedruckt. Die Isolationsschicht 1a, die über die Leiterzüge gedruckt wird, weist über den Elektrodenflächen jeweils rechteckige Aussparungen 2a, 3a, 4a auf, so dass damit die Messfensterflächen der Elektrodenanordnungen 2, 3, 4 definiert werden.

Die drei resultierenden voltammetrischen Drei-Elektrodenanordnungen 2, 3, 4 sind geometrisch identisch. Deren drei Kohlenstoff-Elektroden 18, 18a, 18b, 19, 19a, 19a, 20, 20a, 20b sind jeweils untereinander über elektrisch leitende Kohlenstoffleiterzüge 21, 22, 23 verbunden bzw. parallel geschalten. Die Leiterzüge sind am Ende des Sensorsupports als Kontaktflächenelemente 21a, 22a, 23a ausgebildet, damit der Sensor elektrisch mit einem Glucosemeter kontaktiert werden kann, das die erforderliche Polarisationsspannung bereitstellt und die Signalverarbeitung übernimmt. Eine zusätzliche Kohlenstoffkontaktfläche 24 schaltet das Gerät ein bzw. aus.

Die einzelnen Arbeits-, Referenz- und Gegenelektroden 18, 18a, 18b, 19, 19a, 19b, 20, 20a, 20b sind in Flussrichtung der Probe jeweils nacheinander angeordnet.

Als Glucosemeter dient vorzugsweise ein von der Firma SensLab- Gesellschaft zur Entwicklung und Herstellung bioelektrochemischer Sensoren mbH, Deutschland entwickelter Prototyp mit der Bezeichnung GlucoLine^{R}.

Die Bestimmung von HbA_{1c} in Blutproben erfolgt mit dem erfindungsgemäßen Mikrofluidiksensor wie folgt: Der Sensor wird in das Glucosemeter eingesteckt, so dass sich das Gerät einschaltet und ein Polarisationspotential an der Arbeitselektrode 18, bereitgestellt wird. Der erfindungsgemäße Mikrofluidiksensor wird nach Positionierung im Glucosemeter mit der zu analysierenden Probe in Kontakt gebracht, aufgrund der einzelnen Probeaufbereitungsschritte beim Durchlaufen der Probe in den jeweiligen Kammern werden die erzeugten, zeitlich versetzten Messsignale für ggf. Interferenzen, den Gehalt an Gesamthämoglobin und den Gehalt an nichtglykiertem Hämoglobin oder glykiertem Hämoglon ermittelt und anschließend wird die Menge an glykiertem Hämoglobin berechnet bzw. der realtive Anteil an glykiertem Hämoglobin durch Differenz- und Verhältnisbildung ausgewiesen. Bevorzugt wird ein Bluttropfen, der im einfachsten Fall an einer Fingerbeere zu entnehmen ist, an den Probeaufnahmespalt 6a des Sensors gehalten und aufgrund von Kapillarkraftwirkung sofort in die Probeaufnahmekammer 6 gesaugt. Bei dem Sensor für das indirekte Nachweisverfahren gelangt auf diese Weise auch ein kleiner Teil der Probe in die erste Messkammer 7 und kommt zum Stillstand. Sobald die Probe eine elektrolytische Verbindung zwischen Referenz-, Arbeits- und Gegenelektrode 19a 18a 20a hergestellt hat, wird bei angelegter Polarisationsspannung ein Peakstrom verursacht, der bei Überschreiten eines vorgegebenen internen Schwellwertes eine Triggerung veranlasst, so dass nach einem festgelegten Zeitregime eine Strommessung erfolgt. Der Triggerstrom kann verwendet werden, um ein akustisches Signal im Glucosemeter zu generieren, das dem Anwender das Ende der Probeaufnahme signalisiert. Falls die erste Messkammer 7 einen Mediator aufweist, wird beim Eintritt der Probe, in diese Messkammer 7 auch der Redoxmediator gelöst und durch elektrochemisch aktive Substanzen der Probe anteilig reduziert. Da während des Probetransports über die Probeaufnahmekammer bis in die erste Messkammer keine Hämolyse erfolgt, resultiert der aus der Reoxidation des Redoxfarbstoffes an der Arbeitselektrode 18a verursachte Faradaysche Strom im Wesentlichen aus der Anwesenheit interferierender Substanzen wie Ascorbat, Harnsäure, oder bestimmter Pharmaka, die in Blut enthalten sein können.

Die in der Probeaufnahmekammer 6 befindliche Blutprobe strömt durch den Übergangskanal 8 in die Hämolysekammer 9, in der das darin befindliche Detergenz sofort gelöst wird und damit eine Hämolyse der Erythrozyten verursacht. Die vollständig hämolysierte Blutprobe gelangt über den gegabelten Austrittskanal 10 der Hämolysekammer 9 in einen Kanal 13, der direkt zu der zweiten Messkammer 11 führt und parallel dazu in die Affinitätskammer 12. In der zweiten Messkammer 11 wird wiederum unmittelbar mit deren Befüllung eine Stromtriggerung verursacht und nach einem festgelegten Zeitregime eine Strommessung veranlasst. Der Faradaysche Strom an dieser Arbeitselektrode 18 resultiert zu einem kleinen Teil aus der Oxidation elektrochemisch aktiver Substanzen der Probe und zum weitaus größeren Teil aus dem freigesetzten elektrochemisch aktiven Gesamthämoglobin.

Der Anteil der hämolysierten Probe, der über die Kanalgabelung 10 die Affinitätskammer 12 erreicht hat, überströmt eine Affinitätsmatrix. Dabei erfolgt eine vollständige Bindung des glykierten Hämoglobinanteils. Nach dem Verlassen der Affinitätskammer 12 gelangt die Probe in die dritte Messkammer 14, in der wieder nach einer Stromtriggerung die Strommessung nach einem zeitlich festgelegten Regime erfolgt. Der Faradaysche Strom an dieser Arbeitselektrode 18 resultiert wieder aus dem kleinen Anteil, der durch elektrochemisch aktive Substanzen der Probe verursacht wird, und vor allem aus dem Anteil des nicht glykierten Hämoglobins. Dementsprechend werden im Messkanal nacheinander drei gut voneinander unterscheidbare Messstromverläufe registriert, die jeweils den typischen Verlauf einer chronoamperometischen Messung aufweisen und jeweils auf den vorangegangenen Stromwert aufsetzen. In analoger Weise können durch Integration der Stromwerte über der Zeit die Ladungskurven zur Signalauswertung genutzt werden.

Aus der Differenz zwischen den Messwerten aus der zweiten und ersten Messkammer 11, 7 kann das Gesamthämoglobin bestimmt werden. Aus der Differenz zwischen den Messwerten aus der dritten und ersten Messkammer 14, 7 kann der Anteil des nicht glykierten Hämoglobins gemessen werden und aus der Differenz zwischen den Ergebnissen der beiden Differenzbildungen kann der glykierte Anteil des Hämoglobins ermittelt werden. Parallel dazu kann eine Differenzbildung der Messwerte aus der dritten und zweiten Messkammer 14, 11 erfolgen, so dass der glykierte Hämoglobinanteil direkt ermittelt werden kann und eine zusätzliche Kontrolle in Bezug auf das Grundstromsignal möglich ist. In einer anderen Ausführung, bei der auf eine Messung in der ersten Messkammer 7 verzichtet wird (keine Dotierung mit Redoxmediator), wird aus der Differenzbildung der Messwerte aus der dritten und zweiten Messkammer 14, 11 der glykierte Anteil des Hämoglobins ermittelt. Der mit dem erfindungsgemäßen Sensor bestimmte Gesamtanteil an glykiertem Hämoglobin wird in Bezug zum Gesamthämoglobin gesetzt und als relativer Wert ausgegeben. Über Vergleichsmessungen mit einem Referenzmessverfahren wird der Wert als HbA_{1c}-Anteil kalibriert. Die Messsignalverarbeitung und Messwertanzeige und -speicherung erfolgt durch das Glucosemeter.

Das Verfahren zum direkten Nachweis von glykiertem Hämoglobin ist dadurch gekennzeichnet, dass man den Mikrofluidiksensor in ein adaptiertes Glucosemeter positioniert, mit der zu analysierenden Probe in Kontakt bringt, an den Arbeitselektroden der Dreielektrodenanordnungen ein Polarisationspotential anlegt und aufgrund der einzelnen Probeaufbereitungsschritte beim Durchlaufen der Probe vom Probeeintrittsspalt 6a der Probeaufnahmekammer 6 bis zu den Auslasskanälen 15, 16, 17 in den jeweiligen Kammern die amperometrisch erzeugten, zeitlich versetzten Messsignale für den Gesamtgehalt an Hämoglobin den Gehalt an restlichem, nach der Probeaufbereitung in der Probe noch enthaltenen nichtglykiertem Hämoglobin und die in der dritten Meskammer 14 angereicherte Menge an glykiertem Hämoglobin ermittelt, wobei zur Bestimmung des glykierten Hämoglobinanteils die Differenz zwischen dem in der dritten Messkammer 14 ermitteltem Messwert aus glykiertem Hämoglobin und dem in der zweiten Messkammer 11 ermittelten Messwert aus restlichen, nichtglykiertem Hämoglobin gebildet wird. Durch die Bildung des Quotienten aus diesem Differenzwert und dem Gesamthämoglobinwert, der aus der Messung in der ersten Meskammer 7 resultiert, wird der relative glykierte Hämoglobinanteil ermittelt. Im Detail ist das bevorzugte Verfahren dem Beispiel 3 entnehmbar.

Der Vorteil des erfindungsgemäßen Mikrofluidiksensors liegt in der einfachen Handhabung, in dem geringen erforderlichen Probevolumen und in der Einschrittanwendung, die sich vom Gebrauch von Glucose-Disposables nicht unterscheidet und auch von einem üblichen Glucosemeter ausgelesen wird.

Durch die Nutzung mehrerer, in der Weggeometrie und Weglänge unterschiedlich gestalteter Mikrokanäle, in denen sowohl Probeaufbereitungsschritte als auch Messschritte erfolgen, und die Kombination mit der voltammetrischen Einkanalsensorvorrichtung, die aus drei parallel geschalteten Drei-Elektrodenanordnungen besteht werden zeitlich versetzte quantifizierbare elektrische Messsignale erzeugt. Der Sensor funktioniert jedoch als HbA_{1c}-Einschritt-Assay. Er ist in Kombination mit einem adaptierten Glucosemeter einfach handhabbar, stellt die Messergebnisse in weniger als drei Minuten zur Verfügung, benötigt geringe Probemengen und ist insbesondere für Point of Care-Anwendungen im Home Care-Bereich und in Arztpraxen geeignet. Es steht damit ein einfach zu bedienendes Messsystem zur Verfügung, das nicht auf Laborpersonal beschränkt ist. Das Ergebnis ist ohne Zeitverzug und Probetransport verfügbar. Darüber hinaus ermöglicht die schnelle Verfügbarkeit der Ergebnisse gegebenenfalls eine zeitnahe Auswertung einer Therapie und eine kurzfristige Therapieentscheidung.

Der erfindungsgemäße voltammetrische Sensor zum Nachweis von glykiertem Hämoglobin wird durch nachfolgende Ausführungsbeispiele und Abbildungen näher erläutert.

### Beispiel 1:

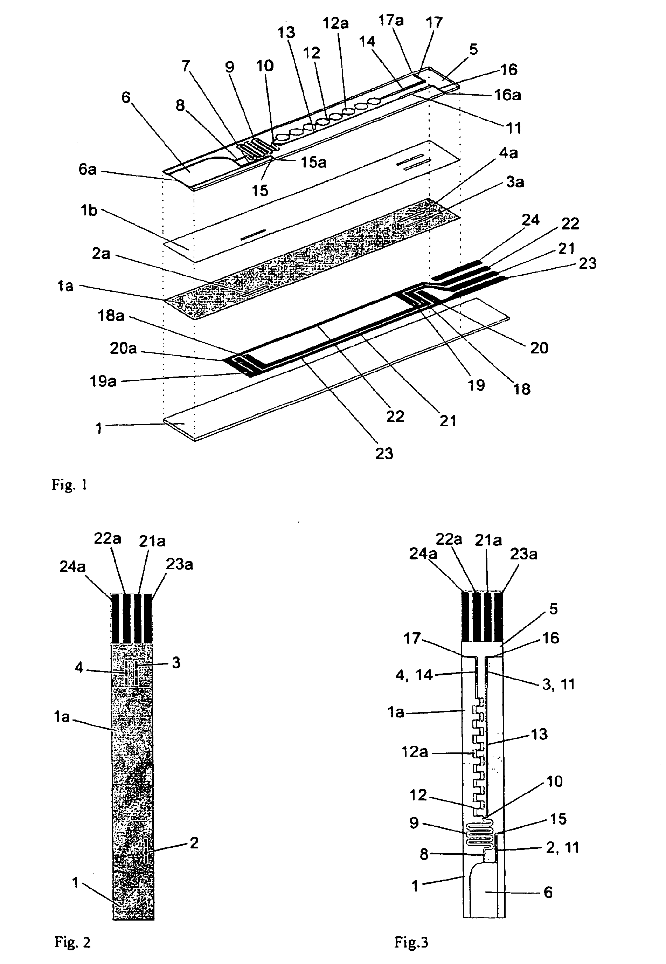

Erfindungsgemäßer voltammetrischer Sensor zum Nachweis von glykiertem Hämoglobin und Gesamthämoglobin. Zur Erläuterung dienen Fig. 1, Fig. 2 und Fig. 4a.

Auf ein planares Trägermaterial 1 aus PET mit einer Dicke von 0,35 mm und Abmessungen von 6 mm x 50 mm werden mittels Siebdruck Elektrodenflächen 18, 18a, 19, 19a, 20, 20a bzw.

Kohlenstoffleiterzüge und -kontaktflächen 21, 21a, 22, 22a, 23, 23a (Acheson PE 401, Acheson NL), und eine Isolationsschicht 1a (240 SB, ESL Europe) wie in Fig. 1 und Fig. 2 dargestellt aufgedruckt und jeweils bei 70°C gehärtet.
Die Geometrie des doppelseitigen Klebefilms 1b ist weitgehend identisch mit der Geometrie der Isolationslackschicht 1 a.
Die Abmessungen eines Sensors sind 6 mm x 40 mm (B x L). Die drei resultierenden voltammetrischen Drei-Elektrodenanordnungen 2, 3, 4 sind geometrisch identisch aufgedruckt. Deren drei Kohlenstoff-Elektroden 18, 18a, 19, 19a, 20, 20a sind jeweils untereinander über elektrisch leitende Kohlenstoffleiterzüge 21, 22, 23 verbunden bzw. parallel geschalten. Die Isolationsschicht 1a, die über die Leiterzüge gedruckt wird, weist über den Elektrodenflächen jeweils rechteckige Aussparungen 2a, 3a, 4a auf, so dass damit die Messfensterflächen der Elektrodenanordnungen 2, 3, 4 definiert werden. Die Leiterzüge sind am Ende des Sensorsupports als Kontaktflächenelemente 21a, 22a, 23a ausgebildet, damit der Sensor elektrisch mit einem Glucosemeter kontaktiert werden kann, das die erforderliche Polarisationsspannung bereitstellt und die Signalverarbeitung übernimmt. Eine zusätzliche Kohlenstoffkontaktfläche 24 schaltet das Gerät ein bzw. aus.
Die einzelnen Arbeits-, Referenz- und Gegenelektroden 18, 18a, 19, 19a, 20, 20a sind in Flussrichtung der Probe jeweils nacheinander angeordnet, wobei die Einzelelektrodenflächen jeweils 0,3 mm² betragen. Auf die Elektrodenanordnungen 2, 3, 4 werden jeweils mit einem Dispenser gleichmäßig verteilt 0,1 µl einer Reaktionslösung pro Elektrodenanordnung aufgetragen, die aus 800 µg Ferricyanid (Sigma), 80 µg Triton-X100 (Sigma) und 50 µg mikrokristalliner Zellulose (Aldrich) besteht. Die resultierende Reaktionsschicht wird 30 min bei 40°C getrocknet.
Mittels eines Stahlformeinsatzes wird eine Polycarbonatfolie von 125 µm Dicke heißgeprägt. Die resultierende Folie weist wie in Fig. 1 dargestellt definierte Mikrostrukturen in Form von Kammern und Kanälen auf, die aus einer Probeaufnahmekammer 6 mit einem Volumen von 3,5 µl und einem Probeaufnahmespalt 6a, einer ersten Messkammer 7, die von der Probeaufnahmekammer 6. direkt abzweigt, einem Übergangskanal 8 von der Probeaufnahmekammer 6 zu einer mäanderförmigen Hämolysekammer 9 mit einem Volumen von 0,4 µl, einem weiteren von der Hämolysekammer 9 abgehenden Austrittskanal 10, der sich gabelt und dessen erster Abzweig über einen Kanal 13 in eine zweite Messkammer 11 und dessen zweiter Abzweig in eine Affinitätskammer 12 mit einem Volumen von 0,8 µl mündet. Die Affinitätskammer 12 führt in eine dritte Messkammer 14. An die drei Messkammern schließen sich Auslasskanäle 15, 16, 17, mit Austrittsöffnungen 15a, 16a, 17a mit jeweils 0,08 µl Volumen an. Das Volumen der Messkammern beträgt jeweils 0,1 µl. Die Tiefen der Kanäle und Kammern betragen zwischen 20 µm und 100 µm. Die Verbindungskanäle sind zwischen 20 µm und 100 µm breit.

In die Probeaufnahmekammer 6 werden mittels Dispenser 1,5 µl einer 0,01 % igen wässrigen Lösung aus Triton-X100, die 0,01 mg Methylenblau enthält über die Fläche gleichmäßig verteilt dosiert. In den Hämolyseraum 9 werden 0,3 µl einer wässrigen 5 % igen Saponinlösung gleichmäßig verteilt eingebracht. Beide Lösungen werden bei 40°C in einem Umlufttrockenschrank getrocknet.

Die Affinitätskammer 12, die aus einer Reihe kreisförmiger und über Kanäle verbundener Einzelkammersegmente 12a mit einem Gesamtvolumen von 0,7 µl besteht, wird mittels einer Zellulosematrix präpariert, die mit Boronsäure modifiziert ist. Zur Herstellung der Matrix werden 100 mg Zellulosepulver (Sigma C-6413) in ein Glasgefäß eingewogen. In dieses Gefäß wird eine wässrige Lösung mit 2mg/ml N-Ethyl-N'-(3-dimethylaminopropyl)carbodiimid (EDC, Sigma E-1769) und 6 mg/ml N-Hydroxysuccinimid (NHS, Sigma H-7377) zugegeben, das Pulver durch schütteln darin suspensiert und für 30 Minuten zur Reaktion gebracht. In diese Suspension wird eine entsprechende Konzentration von 10 mg/ml *m*-Aminophenylboronsäure (Fluka 09199) eingebracht und für weitere 30 Minuten inkubiert. Nach der Reaktion, bzw. Bildung von Säureamidbindungen zwischen Hydroxylgruppen der Cellulose und der Aminogruppe der *m*-Aminophenylboronsäure, wird das Reaktionsgemisch über eine Fritte abgetrennt und die boronsäureimmobilisierte Zellulosematrix mit phosphatgepufferter Saline und Wasser gewaschen. Das aktivierte Pulver wird in 0,1 M Taurinpuffer (Sigma, T-0625) pH 9 aufgenommen und darin gelagert. Diese Lösung enthält zusätzlich 1,5 % Polyethylenglykol (Aldrich 202452), das die Haftung der Zellulosematrix am Kunststoff der Fluidikstrecke verbessert. Von der viskosen aktivierten Zellulosematrix werden dann pro kreisförmigem Kammersegment 0,1 µl eingetropft und bei 40 °C im Durchlauftrockner über 30 min getrocknet.

Schließlich wird die mikrostrukturierte Kunststofffolie 5 mittels einer doppelseitigen, 49 µm dicken Klebefolie (Tesa) mit Grundsensor 1 formschlüssig und irreversibel verbunden. Dabei positionieren sich die Messkammern 7, 11, 14 jeweils unmittelbar über einer der drei Elektrodenanordnungen 2, 3, 4.

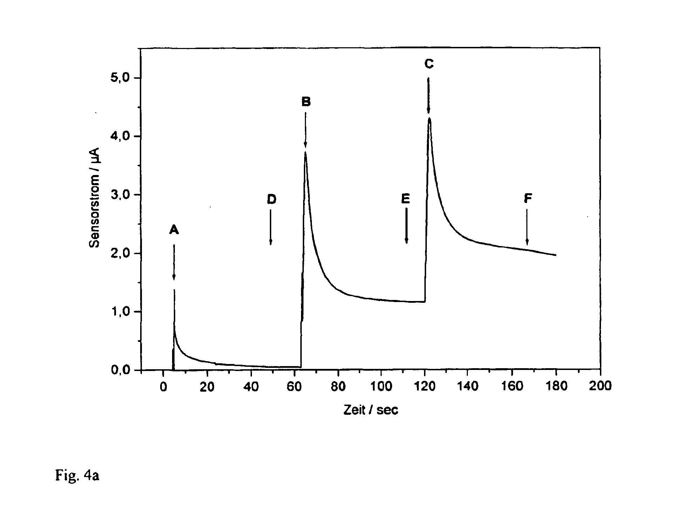

Zur Durchführung einer HbA 1 c - Bestimmung wird der Sensor über die Kontaktflächen 21a, 22a, 23a an das Glucosemeter "GlucoLine" eingesteckt und dadurch die Betriebsbereitschaft des Messgerätes hergestellt. An der Arbeitselektrode 18 wird ein Polarisationspotential von 450 mV bereitgestellt, dass über die Referenzelektrode 19 kontrolliert wird. Der amperometrische Messstrom wird zwischen Arbeits- und Gegenelektrode 18, 20 generiert. Durch Kontakt des Probeaufnahmespalts 6a mit einem Bluttropfen wird aufgrund der Kapillarwirkung die Probe umgehend in die Probeaufnahmekammer 6 gesaugt. Auf diese Weise gelangt auch ein kleiner Teil der Probe in die erste Messkammer 7 und kommt nach Befüllung des Auslasskanals 15 zum Stillstand. Sobald die Probe eine elektrolytische Verbindung zwischen Referenz-, Arbeits-und Gegenelektrode 19, 18, 20 hergestellt hat, wird bei angelegter Polarisationsspannung ein Peakstrom A (Fig. 4a) verursacht, der bei Überschreiten eines vorgegebenen internen Schwellwertes von 300 nA eine Triggerung veranlasst, so dass nach einem festgelegten Zeitregime eine Strommessung zum Zeitpunkt D (Fig. 4a) erfolgt. Da während des Probetransports über die Probeaufnahmekammer bis in die erste Messkammer keine Hämolyse erfolgt, resultiert der aus der Reoxidation des Ferrocyanids an der Arbeitselektrode 18a verursachte Faradaysche Strom im Wesentlichen aus der Anwesenheit interferierender Substanzen wie Ascorbat, Harnsäure, oder bestimmter redoxaktiver Arzneimittel wie Paracetamol, die in Blut enthalten sein können. Der Triggerstrom wird außerdem dazu genutzt, um ein akustisches Signal im Glucosemeter zu generieren, das dem Anwender das Ende der Probeaufnahme signalisiert.

Die in der Probeaufnahmekammer 6 befindliche Blutprobe strömt über den Übergangskanal 8 in die Hämolysekammer 9, in der das darin befindliche Saponin sofort gelöst wird und damit eine Hämolyse der Erythrozyten verursacht. Die vollständig hämolysierte Blutprobe gelangt über den Austrittskanal 10 der Hämolysekammer 9 auf einen sich gabelnden Kanal 10, der über einen Kanal 13 in eine zweite Messkammer 11 führt und parallel dazu in eine Affinitätskammer 12. In der zweiten Messkammer 11 wird wiederum unmittelbar mit deren Befüllung zum Zeitpunkt B (Fig. 4a) eine Stromtriggerung verursacht und nach einem festgelegten Zeitregime eine Strommessung zum Zeitpunkt E veranlasst. Der Faradaysche Strom an dieser Arbeitselektrode 18 resultiert zu einem kleinen Teil aus der Oxidation elektrochemisch aktiver Substanzen der Probe und zum weitaus größeren Teil aus dem freigesetzten elektrochemisch aktiven Gesamthämoglobin.

Der Anteil der hämolysierten Probe, der über die Kanalgabelung 10 die Affinitätskammer 12 erreicht hat, überströmt eine Affinitätsmatrix. Dabei erfolgt eine vollständige Bindung des glykierten Hämoglobinanteils an die exponierten Boronsäuremoleküle. Nach dem Verlassen der Affinitätskammer 12 gelangt die Probe in die dritte Messkammer 14, in der wieder nach einer Stromtriggerung zum Zeitpunkt C (Fig. 4a) die Strommessung nach einem zeitlich festgelegten Regime zum Zeitpunkt F erfolgt. Der Faradaysche Strom an dieser Arbeitselektrode 18 resultiert wieder aus dem kleinen Anteil, der durch elektrochemisch aktive Substanzen der Probe verursacht wird, und vor allem aus dem Anteil des nicht glykierten Hämoglobins. Dementsprechend werden im Messkanal nacheinander drei gut voneinander unterscheidbare Messstromverläufe registriert, die jeweils den typischen Verlauf einer chronoamperometischen Messung aufweisen und jeweils auf den vorangegangenen Stromwert aufsetzen (Fig. 4a).
Aus der Differenz zwischen den Messwerten aus der zweiten und ersten Messkammer 11, 7 wird durch das Glucosemeter das Gesamthämoglobin bestimmt. Aus der Differenz zwischen den Messwerten aus der dritten und ersten Messkammer 14, 7 wird der Anteil des nicht glykierten Hämoglobins ermittelt und aus der Differenz zwischen den Ergebnissen der beiden Differenzbildungen wird schließlich der glykierte Anteil des Hämoglobins ermittelt und auf das Gesamthämoglobin bezogen.
Die resultierenden Werte werden durch das Messgerät nacheinander zur Anzeige gebracht und abgespeichert.

### Beispiel 2:

Erfindungsgemäßer voltammetrischer Sensor zum Nachweis von glykiertem Hämoglobin. Zur Erläuterung dienen Fig. 1, Fig. 3 und Fig. 4b.

Es wird ein voltammetrischer Grundsensor wie in Beispiel 1 beschrieben und in Fig. 2 und Fig. 3 abgebildet hergestellt.
Mittels eines Dispensers werden jeweils 0,1 µl einer Reaktionslösung, die 800 µg Ferricyanid (Sigma), 80 µg Triton-X100 (Sigma) und 50 µg mikrokristalliner Zellulose (Aldrich) enthält über die Elektrodenanordnungen 3, 4 gleichmäßig verteilt aufgetragen. Über die Elektrodenanordnung 2 wird 0,1 µl einer wässrigen Lösung, die 0,1 % Triton-X100 enthält aufgetragen.
Für den Reaktions- und Messkammeraufsatz 5 wird eine Polycarbonatfolie von 250 µm Dicke mittels einer Stahlpatrize heißgeprägt. Die resultierende Folie weist wie in Fig. 3 dargestellt definierte Mikrostrukturen in Form von Kammern und Kanälen auf, die aus einer Probeaufnahmekammer 6 mit Probeaufnahmespalt 6a und einem Volumen von 3,5 µl , einer ersten Messkammer 7, die von der Probeaufnahmekammer 6 direkt abzweigt, einem Übergangskanal 8 von der Probeaufnahmekammer 6 zu einer mäanderförmigen Hämolysekammer 9 mit einem Volumen von 0,8 µl, einem weiteren von der Hämolysekammer 9 abgehenden Austrittskanal 10, der sich gabelt und dessen erster Abzweig in eine zweite Messkammer 11 und dessen zweiter Abzweig in eine Affinitätskammer 12 mit sechzehn rechteckigen Einzelsegmenten und einem Gesamtvolumen von 1,0 µl mündet. Der Ausgang der Affinitätskammer 12 führt zu einer dritten Messkammer 14. Den Messkammern sind jeweils Auslasskanäle 15, 16, 17, mit Austrittsöffnungen 15a, 16a, 17a angeschlossen, deren Volumen jeweils 0,08 µl beträgt. Das Volumen der Messkammern umfasst jeweils 0,1 µl. Die Tiefen der
Kanäle und Kammern betragen zwischen 20 µm und 100 µm. Die Verbindungskanäle sind zwischen 20 µm und 100 µm breit.
In die Probeaufnahmekammer 6 werden mittels Dispenser 1,5 µl einer 0,01 % igen wässrigen Lösung aus Triton-X100, über die geprägte Fläche gleichmäßig verteilt dosiert. In den Hämolyseraum werden 0,3 µl einer wässrigen 5 % igen Saponinlösung gleichmäßig verteilt eingebracht. Beide Lösungen werden bei 40°C in einem Umlufttrockenschrank getrocknet.
Als Affinitätskammer 12 wird eine mäanderförmige Struktur mit wechselnden Kanalbreiten verwendet. Die entlang der Längsachse verlaufenden sechzehn rechteckigen Einzelsegmente des Mäanders weisen jeweils ein Einzelvolumen von 0,06 µl auf und werden mit boronsäuremodifizierten Sepharosegelkügelchen befüllt, die einen Durchmesser zwischen 45 µm bis 165 µm aufweisen.
Zur Herstellung der boronsäureimmobilisierten Sepharosegelkügelchen wird NHS-aktivierte Sepharose 4 Fast Flow (17-0906-01) der Firma Amersham Bioscience verwendet. Die Sepharose wird in definierter Menge in eine Fritte gefüllt und mit der 10 bis 15-fachen Menge kalter 1 mM HCl gewaschen. Im Vergleich zur Sepharosemenge wird in der halben Menge *m*-Aminophenylboronsäure in Wasser gelöst, mit der gewaschenen Sepharose vermischt und zwei Stunden bei Raumtemperatur gekoppelt. Nach der Reaktion werden die noch frei gebliebenen Gruppen für einige Stunden in einem 0,1 M Ethanolaminpuffer geblockt. Anschließend wird die Sepharosematrix mit der dreifachen Menge an 0,1 M Tris-HCl Puffer (pH 8) und drauffolgend mit 0,1 M Acetatpuffer pH 4 gewaschen und das ganze dreimal wiederholt. Zur Befüllung der Einzelsegmente 12a wird die aktivierte Sepharosematrix in 0,1 M Tris/HCl-Puffer pH 9 aufgenommen. Diese Lösung enthält zusätzlich 1,5 % Polyethylenglykol (Aldrich 202452). Pro Einzelsegment werden 0,1 µl (0,06 µl) der Lösung eingebracht. Nach dem Trocknen der aktivierten Sepharosegelkügelchen bei Raumtemperatur wird die derart präparierte mikrostrukturierte Polycarbonatfolie mittels doppelseitiger Klebefolie (Tesa) auf dem Grundsensor formschlüssig und irreversibel verbunden. Dabei positionieren sich die Messkammern 7, 11, 14 jeweils unmittelbar über einer der drei Elektrodenanordnungen 2, 3, 4.

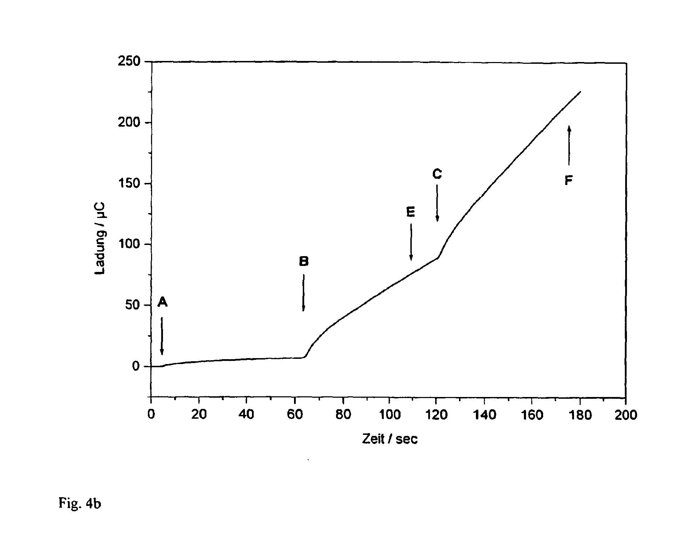

Zur Durchführung einer HbA_{1c} - Bestimmung wird der Sensor über die Kontaktflächen 21a, 22a, 23a an das Glucosemeter "GlucoLine" eingesteckt und dadurch die Betriebsbereitschaft des Messgerätes hergestellt. An der Arbeitselektrode 18 wird ein Polarisationspotential von 450 mV bereitgestellt, dass über die Referenzelektrode 19 kontrolliert wird. Der amperometrische Messstrom wird zwischen Arbeits- und Gegenelektrode 18, 20 generiert. Durch Kontakt des Probeaufnahmespalts 6a mit einem Bluttropfen wird aufgrund der Kapillarwirkung die Probe umgehend in die Probeaufnahmekammer 6 gesaugt. Auf diese Weise gelangt auch ein kleiner Teil der Probe in die erste Messkammer 7 und kommt nach Befüllung des Auslasskanals 15 zum Stillstand. Sobald die Probe eine elektrolytische Verbindung zwischen Referenz-, Arbeits-und Gegenelektrode 19a, 18a, 20a hergestellt hat wird bei angelegter Polarisationsspannung ein Ladungsanstieg A (Fig. 4b) verursacht, der bei Überschreiten eines vorgegebenen internen Schwellwertes von 1 µC eine Triggerung veranlasst, um ein akustisches Signal im Glucosemeter zu generieren, das dem Anwender die Befüllung der Probeaufnahmekammer signalisiert und damit das Ende der Probeaufnahme.
Die in der Probeaufnahmekammer 6 befindliche Blutprobe strömt durch den Übergangskanal 8 in die Hämolysekammer 9, in der das darin befindliche Saponin sofort gelöst wird und damit eine Hämolyse der Erythrocyten verursacht. Die vollständig hämolysierte Blutprobe gelangt über einen sich gabelnden Kanal 10, der der Hämolyskammer folgt, durch einen weiteren Kanal 13 in eine zweite Messkammer 11 und parallel dazu in eine Affinitätskammer 12. In der zweiten Messkammer 11 wird wiederum unmittelbar mit deren Befüllung zum Zeitpunkt B (Fig. 4b) eine Ladungstriggerung verursacht und nach einem festgelegten Zeitregime eine Ladungsmessung zum Zeitpunkt E veranlasst. Der Ladungsumsatz, der an dieser Arbeitselektrode 18 ermittelt wird, resultiert zu einem kleinen Teil aus der Oxidation elektrochemisch aktive Substanzen der Probe und zum weitaus größeren Teil aus dem freigesetzten elektrochemisch aktiven Gesamthämoglobin.
Der Anteil der hämolysierten Probe, der über die Kanalgabelung 10 die Affinitätskammer 12 erreicht hat, überströmt eine Affinitätsmatrix. Dabei erfolgt eine vollständige Bindung des glykierten Hämoglobinanteils an die exponierten Boronsäuremoleküle. Die quer zur Strömungsrichtung angeordneten Verbindungskanäle zwischen den Einzelsegmenten 12a verhindern aufgrund ihres geringen Querschnitts, dass einzelne Sepharosekügelchen austreten können. Darüber hinaus führt die Querschnittsveringerung zu einer Verzögerung des Probeflusses, so dass die Inkubationszeit in den Einzelsegmenten erhöht wird. Nach dem Verlassen der Affinitätskammer 12 gelangt die Probe in die dritte Messkammer 14, in der wieder nach einer Triggerung zum Zeitpunkt C (Fig. 4b) die Ladungsmessung nach einem zeitlich festgelegten Regime zum Zeitpunkt F erfolgt. Der Ladungstransfer an dieser Arbeitselektrode 18 resultiert wieder aus dem kleinen Anteil, der durch elektrochemisch aktive Substanzen der Probe verursacht wird, und vor allem aus dem Anteil des nicht glykierten Hämoglobins. Dementsprechend werden im Messkanal nacheinander zwei gut voneinander unterscheidbare Ladungsverläufe registriert, die jeweils auf den vorangegangenen Wert aufsetzen (Fig. 4b).

Aus der Differenz der Ladungswerte aus der dritten und der zweiten Messkammer 14, 11 wird durch das Glucosemeter der Anteil des glykierten Hämoglobins ermittelt und auf den Gesamthämoglobinanteil bezogen.. Der resultierende relative Wert wird durch das Messgerät zur Anzeige gebracht und abgespeichert. Der mit dem erfindungsgemäßen Sensor bestimmte realtive glykierte Hämoglobinanteil kann als realtiver HbA_{1c}-Anteil standardisiert werden.

### Beispiel 3:

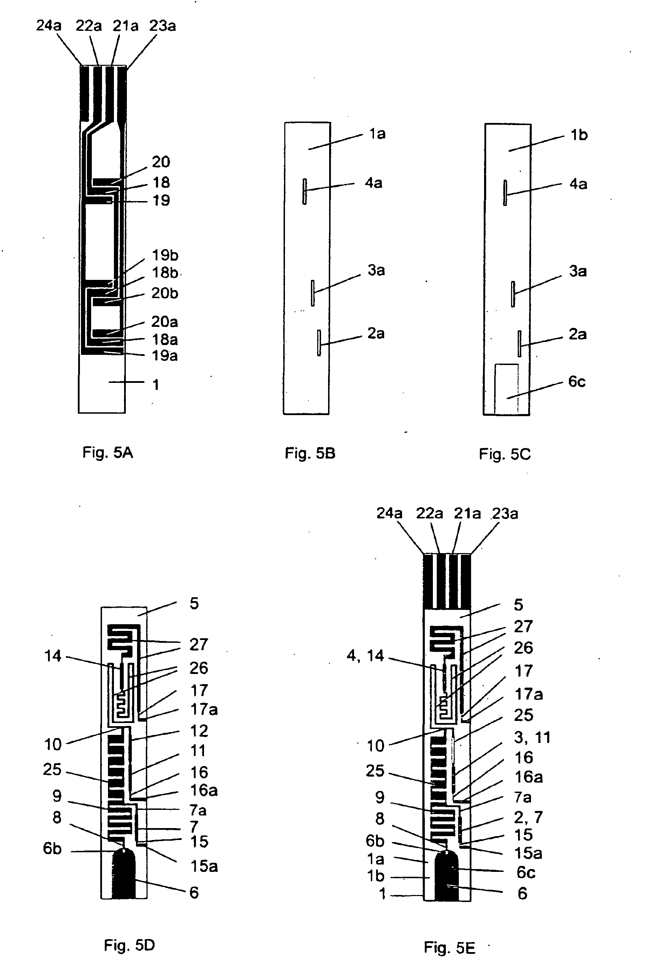

### Erfindungsgemäßer voltammetrischer Sensor zum Nachweis von glykiertem Hämoglobin und Gesamthämoglobin. Zur Erläuterung dienen Fig. 5A bis Fig. 5E.

Auf einen planaren Trägermaterial 1 aus PET mit einer Dicke von 0,35 mm und Abmessungen von 6 mm x 43 mm werden mittels Siebdruck Elektrodenflächen 18, 18a, 19, 19a, 20, 20a, 18b, 19b, 20b bzw. Kohlenstoffleiterzüge - und Kontaktflächen 21, 21a, 22, 22a, 23, 23a (Acheson PE 401, Acheson NL) wie in Abb. 5A dargestellt, und eine Isolationsschicht 1a (240 SB, ESL Europe) wie in Fig. 5B dargestellt aufgedruckt und jeweils bei 70°C gehärtet. Die Isolationsschicht 1a, die über die Leiterzüge gedruckt wird, weist über den Elektrodenflächen jeweils rechteckige Aussparungen 2a, 3a, 4a auf, so dass damit die Messfensterflächen der Elektrodenanordnungen 2, 3, 4 definiert werden (Fig. 5B). Die drei resultierenden voltammetrischen Drei-Elektrodenanordnungen 2, 3, 4 sind geometrisch identisch. Deren drei Kohlenstoff-Elektroden 18, 18a, 18b, 19, 19a, 19a, 20, 20a, 20b sind jeweils untereinander über elektrisch leitende Kohlenstoffleiterzüge 21, 22, 23 verbunden bzw. parallel geschalten (Fig. 5A). Die Leiterzüge sind am Ende des Sensorsupports als Kontaktflächenelemente 21 a, 22a, 23a ausgebildet, damit der Sensor elektrisch mit einem Glucosemeter kontaktiert werden kann, das die erforderliche Polarisationsspannung bereitstellt und die Signalverarbeitung übernimmt. Eine zusätzliche Kohlenstoffkontaktfläche 24 schaltet das Gerät ein bzw. aus. Die einzelnen Arbeits-, Referenz- und Gegenelektroden 18, 18a, 18b, 19, 19a, 19b, 20, 20a, 20b sind in Flussrichtung der Probe jeweils nacheinander angeordnet, wobei die Einzelelektrodenflächen jeweils 0,1 mm² betragen. Die Geometrie des doppelseitigen Klebefilms 1b ist weitgehend identisch mit der Geometrie der Isolationslackschicht 1a, weist jedoch in den Abmaßen (LxB) der Probeaufnahmekammer neben den Aussparungen für die Messfenster 2a,3a, 4a eine zusätzliche Aussparung für die Probeaufnahmekammer 6c auf (Fig. 5C). Mittels eines Stahlformeinsatzes wird eine Polycarbonatfolie von 250 µm Dicke heißgeprägt. Die resultierende Folie weist wie in Fig. 5D schematisch dargestellt definierte Strukturen in Form von Kammern und Kanälen auf. Die Probeaufnahmekammer 6 mit einem Volumen von 4,2 µl verfügt am Ende der Kammer über eine mittig angeordnete, kreisförmigen Öffnung 6b, in deren unmittelbarer Nähe ein Übergangskanal 8 die Probeaufnahmekammer 6 mit einer mäanderförmigen Hämolysestrecke 9 mit einem Volumen von 825 nl verbindet. Am Ende der Hämolysestrecke 9 führt ein Übergangskanal 7a zu einer ersten Messkammer 7. Die Hämolysestrecke 9 geht in eine weitere mäanderförmige Strecke zur Rückhaltung des nichtglykierten Hämoglobins (HbA-Rückhaltestrecke) 25 über, die ein positiv- oder negativ geladenes poröses Trägermaterial enthält und ein Volumen von 1,2 µl aufweist. Von der HbA-Rückhaltestrecke 25 geht ein Kanal 10 ab, der sich gabelt und dessen erster Abzweig über eine Affinitätskammer 12 mit einem Volumen von 84 nl in eine zweite Messkammer 11 und dessen zweiter Abzweig über eine Verzögerungsstrecke 26 mit einem Volumen von 500 nl in eine dritte Messkammer 14 mündet. An zwei der Messkammern 7, 11 schließen sich Auslasskanäle 15, 16 mit jeweils 80 nl Volumen an, die in Austrittsöffnungen 15a, 16a münden. Die dritte Messkammer 14 geht in eine Abfallstrecke 27 über, das ein Volumen von 1,2µl aufweist und der wiederum ein Auslasskanal 16 und eine Austrittsöffnung 16a folgt. Die Geometrien der Messkammern 7, 11, 14 sind identisch und betragen jeweils 0,3 mm x 7 mm x 0,08 mm (84 nl). Die Tiefen der Kanäle und Kammern betragen zwischen 20 µm und 100 µm. Die Verbindungskanäle sind zwischen 20 µm und 100 µm breit. Das Gesamtvolumen der Anordnung, das die Probe von der Probeaufnahmekammer aus beginnend durchströmt, beträgt 4,15 µl.
Auf die Elektrodenanordnungen 2, 3, 4 werden jeweils mit einem Dispenser gleichmäßig verteilt 0,1 µl einer Reaktionslösung pro Elektrodenanordnung aufgetragen, die aus 800 µg Ferricyanid (Sigma), 160 µg Triton-X100 (Sigma) und 50 µg mikrokristalliner Zellulose (Aldrich) besteht. Die resultierende Reaktionsschicht wird 30 min bei 40°C getrocknet. In die Probeaufnahmekammer 6 werden mittels Dispenser 1,5 µl einer 0,1 % igen wässrigen Lösung aus Triton-X100 über die Fläche gleichmäßig verteilt dosiert. In die mäanderförmige Hämolysestrecke 9 werden 0,3 µl einer wässrigen 5 % igen Saponinlösung gleichmäßig verteilt eingebracht. Beide Lösungen werden bei 40°C in einem Umlufttrockenschrank getrocknet. In die rechteckförmigen Einzelsegmente 25a der Hämoglobinrückhaltestrecke 25 mit den Abmaßen (LxBxH) von 2 mm x 1 mm x 0,05 mm werden ausgestanzte Filterpapierabschnitte (3MM Whatman) mit gleichen Abmaßen eingelegt, die mit einer Lösung des Kationenaustauschers Nafion® (Du Pont) behandelt wurden.
In die Messkammer 14 und die Affinitätskammer 26 werden Filterpapierabschnitte mit Abmaßen von 0,3 mm x 7 mm x 0,05 mm eingesetzt, die zuvor mit Boronsäure modifiziert werden. Zur Modifizierung der Zellulosematrix wird das Papier in ein Glasgefäß gelegt. In dieses Gefäß wird eine wässrige Lösung mit 2mg/ml N-Ethyl-N'-(3-dimethylaminopropyl)-carbodiimid (EDC, Sigma E-1769) und 6 mg/ml N-Hydroxysuccinimid (NHS, Sigma H-7377) zugegeben und für die Reaktion unter leichtem Schütteln über 30 Minuten inkubiert. Im Anschluss wird eine entsprechende Konzentration von 10 mg/ml *m*-Aminophenylboronsäure (Fluka 09199) eingebracht und für weitere 30 Minuten unter leichtem Schütteln inkubiert. Nach der Reaktion, bzw. Bildung von Säureamidbindungen zwischen Hydroxylgruppen der Cellulose und der Aminogruppe der *m*-Aminophenylboronsäure, wird die boronsäureimmobilisierte Zellulosematrix nacheinander mit Phosphatpuffer und Wasser gewaschen. Die aktivierte Matrix wird schließlich in 0,1 M Taurinpuffer (Sigma, T-0625) pH 9 getränkt und bei 40 °C über 30 min getrocknet. Danach wird das Filterpapier auf die Rechteckabmasse von 0,3 mm x 7 mm geschnitten und in die Messkammer 14 und Affinitätskammer 12 eingesetzt.
Schließlich wird die mikrostrukturierte Kunststofffolie 5 mittels einer doppelseitigen, 30 µm dicken Klebefolie (3 M), die wie in Fig. 5C dargestellt, Ausstänzungen für die Probeaufnahmekammer 6, und die Messkammern 7, 11, 14 aufweist, mit dem Grundsensor 1 formschlüssig und irreversibel verbunden. Dabei positionieren sich die Messkammern 7, 11, 14 jeweils unmittelbar über einer der drei Elektrodenanordnungen 2, 3, 4.
Zur Durchführung einer HBA1c -Bestimmung wird der Sensor über die Kontaktflächen 21a, 22a, 23a an das Glucosemeter "GlucoLine" eingesteckt und dadurch die Betriebsbereitschaft des Messgerätes hergestellt. An der Arbeitselektrode 18 wird ein Polarisationspotential von 450 mV bereitgestellt, dass über die Referenzelektrode 19 kontrolliert wird. Der amperometrische Messstrom wird zwischen Arbeits- und Gegenelektrode 18, 20 generiert
Durch Kontakt des Probeaufnahmespalts 6a mit einem Bluttropfen wird aufgrund der Kapillarwirkung die Probe umgehend in die Probeaufnahmekammer 6 gesaugt. Die am Ende der Probeaufnahmekammer angeordnete kreisförmige Öffnung 6b beschleunigt die Probeaufnahme erheblich.

Von der Probeaufnahmekammer 6 aus gelangt die Probe über den Verbindungskanal 8 zur Hämolysestrecke 9. Beim Durchströmen der Strecke löst die Blutprobe das darin befindliche Saponin auf und es erfolgt eine umgehende Hämolyse der Erythrozyten, die am Ende der Hämolysestrecke 9 vollständig ist. Durch einen Übergangskanal 7a gelangt ein Teil der hämolysierten Probe in die erste Messkammer 7 und kommt am Ende der Messkammer 7 zum Stillstand, da der Auslasskanal 15 aufgrund seines sehr geringen Querschnitts zwar die verdrängte Luft zur Auslassöffnung 15a passieren lässt, nicht aber die Probeflüssigkeit selbst. Sobald die Probe eine elektrolytische Verbindung zwischen Referenz-, Arbeits- und Gegenelektrode19, 18, 20 hergestellt hat wird bei angelegter Polarisationsspannung ein Peakstrom verursacht, der bei Überschreiten eines vorgegebenen internen Schwellwertes von 300 nA eine Triggerung veranlasst, so dass nach einem festgelegten Zeitregime eine Strommessung zu einem definierten Zeitpunkt D erfolgt. Der resultierende Faradaysche Messstrom an der Arbeitselektrode 18a resultiert im Wesentlichen aus der Reoxidation des Ferrocyanids, das durch das Gesamthämoglobin der Probe reduziert wird.
Der Probehauptstrom gelangt jedoch von der Hämolysestrecke 9 direkt in die HbA-Rückhaltestrecke 25, die aufgrund ihrer mit Kationenaustauscher dotierten Zellulosematrixeinsätze eine Rückhaltung des nichtglykierten Hämoglobins, das positiver als das glykierte Hämoglobin geladen ist, bewirkt.
Nach dem Durchlaufen der HbA-Rückhaltestrecke 25 gelangt die Probe in einen Kanal 10, der sich gabelt. Der erste Gabelabzweig führt über die Affinitätskammer 12 in eine zweite Messkammer 11.

Die mit immobilisierter Boronsäure modifizierte Affinitätskammer 12 bindet das glykierte Hämoglobin, so dass die Probe, die dann in die zweite Messkammer 11 gelangt, gegebenenfalls noch nichtglykiertes Hämoglobin enthält, das beim Durchlaufen der HbA-Rückhaltestrecke 25 nicht gebunden wurde.
In der zweiten Messkammer 11 wird wiederum unmittelbar mit deren Befüllung eine Stromtriggerung verursacht und nach einem festgelegten Zeitregime eine Strommessung zum Zeitpunkt veranlasst. Der Faradaysche Strom an dieser Arbeitselektrode 18 resultiert im Wesentlichen aus dem elektrochemisch aktiven Resten an nichtglykiertem Hämoglobins der Probe.

Über den zweiten Gabelabzweig der Gabelung 10 gelangt der andere Anteil der Probe, in die Verzögerungsstrecke 26, die erforderlich ist, um die Messignalgenerierung zwischen der zweiten und dritten Messkammer 11, 14 zeitlich versetzt voneinander erfassen zu können.
Die Verzögerungsstrecke 26 mündet in der dritten Meskammer 14, dabei überströmt die Probe die mit Boronsäure modifizierte Zellulosematrix, so dass das glykierte Hämoglobin der Probe an die exponierten Boronsäuremoleküle anbinden. Das Probevolumen, das die Messkammer 14 durchströmt, und damit die Dauer der Anreicherung des glykierten Hämoglobins wird durch das Volumen des Probeabfallkanals 27 festgelegt. Erst wenn dieser vollständig gefüllt ist, endet der Probefluss. Der Probeabfallkanal weist ein Volumen von 1,2 µl auf. Die Probe kommt wiederum aufgrund der Geometrie des Auslasskanals 17 zum Stillstand.
Durch die Anreicherung des glykierten Hämoglobins wird das Konzentrationsverhältnis in der von glykiertem zu restlichem nichtglykiertem Hämoglobin der Probe in der dritten Messkammer 14 wesentlich verbessert und damit der interferierende Einfluss des nichtglykierten Hämoglobinrestes der Probe bei der direkten Messung des glykierten Anteils vermindert.
Die Strommessung in der dritten Messkammer 14, erfolgt wieder nach einer Stromtriggerung zu einem definierten Zeitpunkt.
Der Faradaysche Strom an dieser Arbeitselektrode 18 resultiert vor allem aus dem Anteil des angereicherten glykierten Hämoglobins der Probe und gegebenenfalls aus einem kleinen Anteil von nichtglykiertem Hämoglobin, das die Hämoglobinrückhaltestrecke passieren konnte.
Dementsprechend werden im Messkanal nacheinander drei gut voneinander unterscheidbare Messstromverläufe registriert, die jeweils den typischen Verlauf einer chronoamperometrischen Messung aufweisen und jeweils auf den vorangegangenen Stromwert aufsetzen.

Aus dem Messwert der ersten Messkammer wird das Gesamthämoglobin ermittelt.

Aus der Differenz zwischen den Messwerten aus der dritten und zweiten Messkammer 14, 11 wird der absolute Anteil des glykierten Hämoglobins ermittelt.
Aus dem Verhältnis der ermittelten Werte für das Gesamthämoglobin und für das glykierte Hämoglobin wird der relative Anteil des glykierten Hämoglobins der Probe berechnet, das über Referenzmessungen und mit definierten Proben an HbA1c auf den relativen HbA1c-Wert kalibriert wird.

Die resultierenden Werte werden durch das Messgerät nacheinander zur Anzeige gebracht und abgespeichert.

### Abbildungen

- Fig. 1: Dreidimensionale Explosivdarstellung eines voltammetrischen Mikrofluidiksensors zum Nachweis von glykiertem Hämoglobin bestehend aus einem planaren Trägermaterial 1, der dreifach und geometrisch identisch aufgedruckt und parallel geschaltete Drei-Elektrodenanordnungen 2, 3, 4 mit jeweils einer Arbeits-, Referenz- und Gegenelektrode 18, 18a, 19, 19a, 20, 20a, sowie Kohlenstoffleiterzüge 21, 22, 23, Isolationslack 1a mit Messfensteröffnungen 2a, 3a, 4a, gemeinsame Kontaktflächen 21a, 22a, 23a und einen zusätzlichen Geräteeinschaltkontakt 24 aufweist, einer doppelseitigen Klebefolie 1b und einer mikrostrukturierten Kunststofffolie 5, in die eine Probeaufnahmekammer 6, eine erste Messkammer 7, ein Übergangskanal 8, eine Hämolysekammer 9, einen sich gabelnden Kanal 10, eine zweite Messkammer 11 eine Affinitätssäule 12 mit kreisförmigen und durch Kanäle verbundenen Einzelsegmenten 12a, einen Kanal 13, der den sich gabelnden Kanal 10 mit der zweiten Messkammer 11 verbindet, eine dritte Messkammer 14 und in die jeweils an den Messkammern sich anschließende Austrittskanäle 15, 16, 17 mit Austrittsöffnungen 15a, 15b, 15c eingeprägt sind.
- Fig. 2: Draufsicht auf den Träger 1 eines voltammetrischen Mikrofluidiksensors zum Nachweis von glykiertem Hämoglobin mit drei Drei-Elektrodenanordnungen 2, 3, 4 Arbeits-, Referenz- und Gegenelektroden 18, 18a, 19, 19a, 20, 20a, Kontaktflächen 21a, 22a, 23a und Schaltkontakt 24 und Isolationslack 1a mit Messfensteröffnungen 2a, 3a, 4a.
- Fig. 3: Draufsicht auf einen voltammetrischen Mikrofluidiksensor zum Nachweis von glykiertem Hämoglobin mit einem planaren Träger 1, Kontaktflächen 21a, 22a, 23a, einem zusätzlichen Geräteeinschaltkontakt 24 und einer mikrostrukturierten Kunststofffolie 5, in die eine Probeaufnahmekammer 6, eine erste Messkammer 7, ein Übergangskanal 8, eine Hämolysekammer 9, einen sich gabelnden Kanal 10, eine zweite Messkammer 11, eine mäanderförmige Affinitätssäule 12 mit rechteckigen Einzelsegmenten 12a, die durch Kanäle verbunden sind, einen Kanal 13, der den sich gabelnden Kanal 10 mit der zweiten Messkammer 11 verbindet, eine dritte Messkammer 14 und in die jeweils an den Messkammern sich anschließende Austrittskanäle 15, 16, 17 eingeprägt sind.
- Fig. 4a,b: Messstromaufzeichnung (Fig. 4a) und Ladungsaufzeichnung (4b) der Messung mit dem voltammetrischen Mikrofluidiksensor zum Nachweis von glykiertem Hämoglobin, wobei die Peakströme A, B und C in Fig. 4a bzw. die Ladungsanstiege A, B, C in Fig. 4b Indikatoren der vollständigen Befüllung der Messkammern bzw. der Bedeckung der Drei-Elektrodenanordnungen mit Probeflüssigkeit und gleichzeitig Triggersignale für definierte Messwertnahmen zu den Zeitpunkten D, E, F sind.
- Fig. 5A: Draufsicht Elektrodenflächen 18, 18a, 19, 19a, 20, 20a, 18b, 19b, 20b bzw. Kohlenstoffleiterzüge - und Kontaktflächen 21, 21 a, 22, 22a, 23, 23a auf Träger 1
- Fig. 5B,: Isolationsschicht 1a, die über die Leiterzüge gedruckt wird, weist über den Elektrodenflächen jeweils rechteckige Aussparungen 2a, 3a, 4a auf
- Fig. 5C: Doppelseitige Klebefolie 1b mit jeweils rechteckigen Aussparungen 2a, 3a, 4a für Elektrodenanordnungen 2 ,3 ,4 und Aussparung 6c für die Probeaufnahme kammer 6.
- Fig. 5D: Schematisch dargestellte definierte Strukturen der heißgeprägten Kunststofffolie 5 in Form von Kammern und Kanälen.
- Fig. 5E: Draufsicht auf Gesamtansicht des Sensors mit bedrucktem Träger 1, doppelseitiger Klebefolie 1b und heißgeprägter Kunststofffolie 5 mit definierten Strukturen in Form von Kammern und Kanälen.

### Bezugszeichenliste

- 1: planarer Träger
- 1 a: Isolationsschicht
- 1b: Klebefolie
- 2, 3, 4: Elektrodenanordnungen
- 2a,3a,4a: Messfensteröffnungen
- 5: Kunsstofffolie
- 6: Probeaufnahmekammer
- 6a: Probeaufnahmekammerspalt
- 6b: kreisförmige Öffnung
- 6c: Aussparung der Klebefolie für Probeaufnahmekammer
- 7: erste Messkammer
- 8: Übergangskanal
- 9: Hämolysekammer
- 10: Austrittskanal
- 11: zweite Messkammer
- 12: Affinitätskammer
- 13: Kanal
- 14: dritte Messkammer
- 15,16, 17: Auslasskanäle
- 15a,16a,17a: Austrittsöffnungen
- 18, 18a: Arbeitselektrode
- 19, 19a: Referenzelektrode
- 20, 20a: Gegenelektrode
- 21,22,23: Kohlenstoffleiterzüge
- 21a,22a,23a: Kontaktflächen
- 24: Geräteeinschaltkontakt
- 25: Fließstrecke (Rückhaltestrecke für nichtglykiertes Hb)
- 26: Verzögerungsstrecke
- 27: Probeabfallkanal

## Patentansprüche

1. Voltammetrischer Mikrofluidiksensor zum direkten oder indirekten Nachweis von glykiertem Hämoglobin wobei
- ein planares Trägermaterial (1), das drei geometrisch identisch aufgedruckte und parallel geschaltete voltammetrische Drei-Elektrodenanordnungen aufweiset, die jeweils aus Arbeits-, Referenz- und Gegenelektroden bestehen,
- eine darüber befindliche Isolationsschicht (1a) mit Messfenstern über den jeweiligen Elekrodenanordnungen und
- eine mikrostrukturierte Kunststofffolie (5), umfassend eine Probeaufnahmekammer (6), drei Messkammern (7), (11) und (14), eine Hämolysekammer (9) zur Hämolyse von Erythrozyten und mindestens eine Affinitätskammer (12) zur Bindung von glykiertem Hämoglobin, sowie Übergangs- und Austrittskanäle, wobei die Reaktions- und Messkammem (6), (7), (9), (11), (12), (14) mit Reagenzien zur Zelllyse, Separation, Affinitätsreaktion und chemischen, elektrochemischen oder enzymatischen Reaktionen dotiert sind,
und wobei planarer Träger (1), Isolationsschicht (1a) und Folie (5) formschlüssig und irreversibel so miteinander verbunden sind, dass die Probeaufnahmekammer (6) in der Folie (5) mit dem Träger (1) einen Spalt (6a) zur Probenaufnahme bildet und die Messkammem jeweils über den Elektrodenanordnungen auf dem Trägermaterial (1) positioniert sind.

2. Mikrofluidiksensor zum direkten Nachweis von glykiertem Hämoglobin nach Anspruch 1, **dadurch gekennzeichnet, dass** die mikrostrukturierte Kunststofffolie (5) umfasst:
die Probeaufnahmekammer (6), die mit der Hämolysekammer (9) verbunden ist, die Hämolysekammer (9), die ein Detergenz zur Hämolyse von Erythrocyten aufweist,
einmal mit der ersten Messkammer (7) die mit einem Redoxmediator zur Ermittlung des Gesarnthämoglobingehaltes der Probe dotiert ist, verbunden ist, und weiterhin über einen Fließabschnitt (25), der so dotiert ist, dass nichtglykiertes Hämoglobin gebunden oder in seinem Fluss merklich behindert wird, mit der Affinitätskammer (12) verbunden ist, die mit mindestens einem affinen Agens zur Bindung von glykiertern Hämoglobin dotiert ist, und an die sich die zweite Messkammer (11), die mediatordotiert zur Ermittlung von restlichem nichtglykiertem Hämoglobin ist, anschließt, wobei ein sich gabelnder Kanal (10) im Anschluss an den Fließabschnitt (25) zum einen die Verbindung zur Affinitätskammer (12) gewährleistet und zum anderen über eine Verzögerungsstrecke (26) mit der dritten Messkammer (14) verbunden ist, die ebenfalls ein affines Agens zur Bindung von glykierten Hämoglobin enthält und mediatordotiert zur Ermittlung von glykiertem Hämoglobin ist, wobei sich an diese Messkammer (14) ein Probeabfallkanal (27) zur Anreicherung von glykiertem Hämoglobin in der dritten Meskammer 14 anschließt.

3. Mikrofluidiksensor nach Anspruch 1 oder 2, **dadurch gekennzeichnet, dass** die jeweils in den Messkammem dotierten Redoxmediatoren und Additive in der Art und Konzentration gleich sind.

4. Mikrofluidiksensor nach einem der Ansprüche 1 bis 3, **gekennzeichnet durch**
a. Trägermaterial (1), das dreifach und geometrisch identisch aufgedruckt parallel geschaltete voltammetrische Drei-Elektrodenanordnungen (2), (3) und (4) aufweist, die jeweils aus Arbeits-, Referenz- und Gegenelektroden (18, 18a), (19, 19a), (20, 20a) bestehen und über Leiterzüge mit Kontaktflächen (21, 21a, 22, 22a, 23, 23a) verbunden sind,
b. Isolationslackschicht (1a),
c. mikrostrukturierte Kunststofffolie (5) enthaltend
i. die Probeaufnahmekammer (6) mit Antidotmittel, an deren Ende sich eine kreisförmige Öffnung (6b) befindet, die in einen
ii. Übergangskanal (8) führt, an den sich die Hämolysekammer (9) mit hämolytischem Detergenz anschließt,
iii. eine aus der Hämolysekammer (9) führenden Kanal (7a), der zur ersten mediatorhaltigen Messkammer (7) führt,
iv. einen aus der Hämolysekammer (9) führenden Fließabschnitt (25), ggf. mit Einzelsegmenten mit affinem Agens,
v. den sich gabelnden Kanal (10), der einmal zur Affinitätskammer (12) führt, ggf mit Einzelsegmenten mit affinem Agens, der die zweite mediatorhaltige Messkammer (11) folgt, und der zum anderen
vi. zur Verzögerungsstrecke (26) führt, der die dritte mediatorhaltige Messkammer mit affinen Agens gegen glykiertes Hämoglobin (14) mit Probeabfallkanal (27) folgt,
vii. jeweils an die Messkammern sich anschließende Austrittskanäle (15), (16), (17) mit Austrittsöffnungen (15a) ,(15b), (15c).

5. Mikrofluidiksensor zum indirekten Nachweis von glykiertem Hämoglobin nach Anspruch 1, **dadurch gekennzeichnet, dass** die mikrostrukturierte Kunststofffolie (5) umfasst:
die Probeaufnahmekammer (6) mit mindestens einem Antidotmittel zur Reduktion von Methämoglobin, an die sich die Hämolysekammer (9) mit mindestens einem Detergenz zur Hämolyse von Erythrozyten anschließt, die zum einen mit der Messkammer (11) verbunden ist, die mit mindestens einem Redoxmediator zur Ermittlung des Gesamt-Hb dotiert ist und zum anderen mit der Affinitätskammer (12),
die mit mindestens einem affinen Agens zur Bindung von glykiertem Hämoglobin dotiert ist, an die sich dann die weitere, ebenfalls mediatordotierte Messkammer (14) zur Ermittlung des nichtglykierten Hämoglobins anschließt, und
dass die Messkammer (7), die sich parallel zur Hämolysekammer (9) an der Probeaufnahmekammer (6) befindet, zur Ermittlung von Interferenzen durch anwesende elektrochemische Substanzen mit mindestens einem Redoxmediator, dotiert ist.

6. Mikrofluidiksensor nach Anspruch 5, **dadurch gekennzeichnet, dass** die jeweils in den Messkammern dotierten Redoxmediatoren und Additive in der Art und Konzentration gleich sind.

7. Mikrofluidiksensor nach einem der Ansprüche 1, 5 oder 6, **gekennzeichnet durch**
a. Trägermaterial (1), das dreifach und geometrisch identisch aufgedruckt parallel geschaltete voltammetrische Drei-Elektrodenanordnungen (2), (3) und (4) aufweist, die jeweils aus Arbeits-, Referenz- und Gegenelektroden (18, 18a), (19, 19a), (20, 20a) bestehen und über Leiterzüge mit Kontaktflächen (21, 21a, 22, 22a, 23, 23a) verbunden sind,
b. Isolationslackschicht (1a),
c. mikrostrukturierte Kunststofffolie (5) enthaltend
i. Probeaufnahmekammer (6) mit Antidotmittel,
ii. die erste mediatorhaltige Messkammer (7), parallel dazu
iii. Übergangskanal (8) mit sich anschließender Hämolysekammer (9) mit hämolytischem Detergenz,
iv. aus der Hämolysekammer (9) führender und sich gabelnder Kanal (10), der über
v. einen direkten Kanal (13) zur zweiten mediatorhaltigen Messkammer (11) führt und parallel
vi. zur Affinitätskammer (12), ggf. mit Einzelsegmenten (12a) mit affinem Agens, der eine dritte mediatorhaltige Messkammer (14) folgt, und
vii. jeweils an die Messkammern sich anschließende Austrittskanäle (15), (16), (17) mit Austrittsöffnungen (15a) ,(15b), (15c).

8. Mikrofluidiksensor nach einem der Ansprüche 1 bis 7, **dadurch gekennzeichnet, dass** die Kunststofffolie (5) eine Dicke von 100 µm bis 500 µm aufweist.

9. Mikrofluidiksensor nach einem der Ansprüche 1 bis 8, **dadurch gekennzeichnet, dass** die Mikrostrukturen in der Kunststofffolie (5) in Form von Kammern und Kanälen Tiefen zwischen 20 µm und 100 µm und Breiten bzw. Längen zwischen 20 µm und 4000 µm aufweisen.

10. Mikrofluidiksensor nach einem der Ansprüche 1 bis 9, **dadurch gekennzeichnet, dass** das Volumen der Probeaufnahmekammer (6) zwischen 1 µl und 10 µl, vorzugsweise 2 µl bis 5 µl, beträgt und das Gesamtvolumen aller anderen Kammern und Verbindungskanäle nicht größer als das der Probeaufnahmekammer (6) ist.

11. Mikrofluidiksensor nach einem der Ansprüche 1 bis 10, **dadurch gekennzeichnet, dass** er zwischen Isolationsschicht (1a) und mikrostrukturierter Kunststofffolie (5) eine Klebefolie (1b) mit Messfenstem über den jeweiligen Elekrodenanordnungen und einer Aussparung 6c über der Probeaufnahmekammer 6 aufweist.

12. Mikrofluidiksensor nach Anspruch 11, **dadurch gekennzeichnet, dass** die Klebefolie (1b) eine Dicke von 30 µm bis 50 µm besitzt und doppelseitig mit dem planaren Träger (1) und der Kunststofffolie (5) nahezu formschlüssig verbunden ist.

13. Mikrofluidiksensor nach einem der Ansprüche 1 bis 12, **dadurch gekennzeichnet, dass** die Kunststofffolie (5) aus Polycarbonat oder Polyester besteht.

14. Mikrofluidiksensor nach einem der Ansprüche 1 bis 13, **dadurch gekennzeichnet, dass** er als Antidotmittel in der Probeaufnahmekammer (6) einen chinoiden Redoxfarbstoff, vorzugsweise Toluidinblau oder Methylenblau in einer Menge zwischen 0,1 mg und 1 mg, aufweist.

15. Mikrofluidiksensor nach einem der Ansprüche 1 bis 14, **dadurch gekennzeichnet, dass** er in der Hämolysekammer (9) als hämolytisch wirksames Detergenz Saponin oder ein nichtionogenes Tensid aufweist, vorzugsweise Saponin in einer Menge zwischen 0,05 mg und 1 mg.

16. Mikrofluidiksensor nach einem der Ansprüche 1 bis 15, **dadurch gekennzeichnet, dass** er als affines Agens in den Affinitätseinrichtungen (12) und (26) Boronsäure, ein Boronsäurederivat oder Antikörper in einer Menge zwischen 0,5 mg und 10 mg aufweist.

17. Mikrofluidiksensor nach einem der Ansprüche 1 bis 14, **dadurch gekennzeichnet, dass** er als Redoxmediator in den jeweiligen Messkammern ein Chinon, Chinonderivat, einen chinoiden Redoxfarbstoff aus der Gruppe der Phenazine, Phenoxazine, Phenothiazine, Indamine, Metallocene, oder einen redoxaktiven Metallkomplex in einer Menge zwischen 0,5 mg und 10 mg aufweist.

18. Mikrofluidiksensor nach einem der Ansprüche 1 und 5 bis 14, **dadurch gekennzeichnet, dass** er in der Fließstrecke (25) als affines Agens zur Bindung von nichtglykiertem Hämoglobin ein geladenes poröses Trägermaterial in Form eines geladenen Polymers oder Ionenaustauschers aufweist.

19. Verfahren zum indirekten Nachweis von glykiertem Hämoglobin **dadurch gekennzeichnet, dass** man einen Mikrofluidiksensor gemäß einem der Ansprüchen 1 bis 4 und 8 bis 17 in ein adaptiertes Glucosemeter positioniert, mit einer zu analysierenden Probe in Kontakt bringt, an die Arbeitselektroden einer potentiostatischen Drei-Elektrodenanordnung, ein Polarisationspotential anlegt und aufgrund der einzelnen Probeaufbereitungsschritte beim Durchlaufen der Probe in den jeweiligen Messkammern, die amperometrisch erzeugten, zeitlich versetzten Strommesssignale für ggf. Interferenzen, den Gesamtgehalt an Hämoglobin und den Gehalt an nichtglykiertem Hämoglobin ermittelt und anschließend den relativen Gehalt an glykiertem Hämoglobin durch Differenzbildung zwischen den Messignalen aus dem Gehalt an Gesamthämoglobin und nichtglykiertem Hämoglobin und der Verhältnisbildung aus der Differenz zum Gesamthämoglobinwert berechnet.

20. Verfahren zum direkten Nachweis von glykiertem Hämoglobin **dadurch gekennzeichnet, dass** man einen Mikrofluidiksensor gemäß einem der Ansprüche 1 und 5 bis 18 in ein adaptiertes Glucosemeter positioniert, mit einer zu analysierenden Probe in Kontakt bringt, an die Arbeitselektroden einer potentiostatischen Drei-Elektrodenanordnung, ein Polarisationspotential anlegt und aufgrund der einzelnen Probeaufbereitungschritte beim Durchlaufen der Probe von der Aufnahme am Kapillarspalt 6a der Probeaufnahmekammer 6 bis zu den Auslasskanälen 15, 16 17 in den jeweiligen Messkammem die amperometrisch erzeugten, zeitlich versetzten Strommesssignale für den Gesamtgehalt an Hämoglobin, den Gehalt an restlichem, nach Probeaufbereitung noch in der Probe enthaltenen nichtglykiertem Hämoglobin und den Gehalt an glykiertem Hämoglobin nach seiner Anreicherung in der dritten Messkammer 14 ermittelt, wobei zur Bestimmung des relativen glykierten Hämoglobinanteils die Differenz zwischen dem in der dritten Messkammer 14 ermitteltem Messwert aus glykiertem Hämoglobin und dem in der zweiten Messkammer 11 ermittelten Messwert aus restlichen, nichtglykiertem Hämoglobin gebildet und zum Gesamthämoglobinanteil, dessen Wert in der ersten Messkammer 7 ermittelt wurde, in Verhältnis gesetzt wird.

## Claims

1. A voltammetric microfluidic sensor for direct or indirect detection of glycated hemoglobin, **characterized by**
- a planar support material (1) having three parallel-connected voltammetric three-electrode arrays geometrically identically printed thereon, each one consisting of working, reference and counter-electrodes,
- an insulating layer (1a) situated thereabove, with measurement windows over the respective electrode arrays, and
- a microstructured plastic film (5) comprising a sample-receiving chamber (6), three measurement chambers (7), (11) and (14), a hemolysis chamber (9) for hemolysis of erythrocytes and at least one affinity chamber (12) for binding glycated hemoglobin, as well as transfer and outlet channels, said reaction and measurement chambers (6), (7), (9), (11), (12), (14) being doped with reagents for cell lysis, separation, affinity reaction, and chemical, electrochemical or enzymatic reactions,
and wherein said planar support (1), insulation layer (1a) and film (5) are positively and irreversibly bonded together in such a way that the sample-receiving chamber (6) in the film (5) forms a gap (6a) with the support (1) for sample uptake, and the measurement chambers are positioned respectively above the electrode arrays on the support material (1).

2. The microfluidic sensor for direct detection of glycated hemoglobin according to claim 1, **characterized in that** the microstructured plastic film (5) comprises:
the sample-receiving chamber (6) connected to the hemolysis chamber (9),
the hemolysis chamber (9) which has a detergent for erythrocyte hemolysis and is connected, on the one hand, to the first measurement chamber (7) doped with a redox mediator to determine the total hemoglobin content of the sample and, on the other hand, via a flow section (25), which is doped in such a way that non-glycated hemoglobin is bound or significantly impeded in its flow, to the affinity chamber (12) doped with at least one affinity agent for binding glycated hemoglobin, which is followed by the second measurement chamber (11) doped with mediator for determining residual non-glycated hemoglobin, a bifurcating channel (10) downstream of flow section (25) on the one hand ensuring connection to the affinity chamber (12) and on the other hand being connected via a delay section (26) to the third measurement chamber (14) which likewise contains an affinity agent for binding glycated hemoglobin and is doped with mediator for determining glycated hemoglobin, said measurement chamber (14) being followed by a sample waste channel (27) for accumulation of glycated hemoglobin in the third measurement chamber 14.

3. The microfluidic sensor according to claim 1 or 2, **characterized in that** the redox mediators and additives respectively doped in the measurement chambers are identical in type and concentration.

4. The microfluidic sensor according to any of claims 1 to 3, **characterized by**
a. a support material (1) having parallel-connected voltammetric three-electrode arrays (2), (3) and (4) printed thereon in triplicate so as to be geometrically identical, each one consisting of working, reference and counter-electrodes (18, 18a), (19, 19a), (20, 20a) and being connected to contact surfaces (21, 21a, 22, 22a, 23, 23a) via conductor tracks,
b. an insulating varnish layer (1a),
c. a microstructured plastic film (5) including
i. the sample-receiving chamber (6) with antidote agent, at the end of which a circular opening (6b) is situated which leads into
ii. the transfer channel (8) which is followed by the hemolysis chamber (9) with hemolytic detergent,
iii. a channel (7a) leading out of the hemolysis chamber (9) and into the first measurement chamber (7) containing mediator,
iv. a flow section (25) leading out of the hemolysis chamber (9), optionally having single segments with affinity agent,
v. the bifurcating channel (10) which on the one hand leads to the affinity chamber (12) optionally having single segments with affinity agent, followed by the second measurement chamber (11) containing mediator, and on the other hand leads to
vi. the delay section (26), followed by the third mediator-containing measurement chamber with affinity agent for glycated hemoglobin (14) with sample waste channel (27),
vii. outlet channels (15), (16), (17) downstream of the respective measurement chambers, with outlet openings (15a), (15b), (15c).

5. The microfluidic sensor for indirect detection of glycated hemoglobin according to claim 1, **characterized in that** the microstructured plastic film (5) comprises:
the sample-receiving chamber (6) with at least one antidote agent for reduction of methemoglobin, which is followed by the hemolysis chamber (9) with at least one detergent for hemolysis of erythrocytes, which is connected, on the one hand, to the measurement chamber (11) doped with a redox mediator to determine the total Hb and, on the other hand, to the affinity chamber (12) doped with at least one affinity agent for binding glycated hemoglobin, which is followed by the additional, likewise mediator-doped measurement chamber (14) for determining non-glycated hemoglobin, and **in that** the measurement chamber (7), which is situated parallel to the hemolysis chamber (9) on the sample-receiving chamber (6), is doped with at least one redox mediator to identify interferences caused by the presence of electrochemical substances.

6. The microfluidic sensor according to claim 5, **characterized in that** the redox mediators and additives respectively doped in the measurement chambers are identical in type and concentration.

7. The microfluidic sensor according to any of claims 1, 5 or 6, **characterized by**
a. a support material (1) having parallel-connected voltammetric three-electrode arrays (2), (3) and (4) printed thereon in triplicate so as to be geometrically identical, each one consisting of working, reference and counter-electrodes (18, 18a), (19, 19a), (20, 20a) and being connected to contact surfaces (21, 21a, 22, 22a, 23, 23a) via conductor tracks,
b. an insulating varnish layer (1a),
c. a microstructured plastic film (5) including
i. the sample-receiving chamber (6) with antidote agent,
ii. the first mediator-containing measurement chamber (7),
iii. the transfer channel (8) parallel thereto, followed by the hemolysis chamber (9) with hemolytic detergent,
iv. the bifurcating channel (10) which leads out of the hemolysis chamber (9) and via
v. a direct channel (13) to the second mediator-containing measurement chamber (11) and is parallel to
vi. the affinity chamber (12) optionally having single segments (12a) with affinity agent, followed by a third mediator-containing measurement chamber (14), and
vii. outlet channels (15), (16), (17) downstream of the respective measurement chambers, with outlet openings (15a), (15b), (15c).

8. The microfluidic sensor according to any of claims 1 to 7, **characterized in that** the plastic film (5) has a thickness of from 100 µm to 500 µm.

9. The microfluidic sensor according to any of claims 1 to 8, **characterized in that** the microstructures in the plastic film (5) in the form of chambers and channels have depths between 20 µm and 100 µm and widths or lengths between 20 µm and 4000 µm.

10. The microfluidic sensor according to any of claims 1 to 9, **characterized in that** the volume of the sample-receiving chamber (6) is between 1 µl and 10 µl, preferably 2 µl to 5 µl, and that the total volume of all the other chambers and connecting channels is not greater than that of the sample-receiving chamber (6).

11. The microfluidic sensor according to any of claims 1 to 10, **characterized in that**, between the insulation layer (1a) and the microstructured plastic film (5), it has an adhesive film (1b) with measurement windows above the respective electrode arrays and a recess 6c above the sample-receiving chamber 6.

12. The microfluidic sensor according to claim 11, **characterized in that** the adhesive film (1b) has a thickness of 30 µm to 50 µm and a virtually positive double-side bond to the planar support (1) and the plastic film (5).

13. The microfluidic sensor according to any of claims 1 to 12, **characterized in that** the plastic film (5) is made of polycarbonate or polyester.

14. The microfluidic sensor according to any of claims 1 to 13, **characterized in that** it has a quinoid redox dye, preferably toluidine blue or methylene blue in an amount between 0.1 mg and 1 mg as antidote agent in the sample-receiving chamber (6).

15. The microfluidic sensor according to any of claims 1 to 14, **characterized in that** it has saponin or a nonionic surfactant as hemolytically active detergent in the hemolysis chamber (9), preferably saponin in an amount between 0.05 mg and 1 mg.

16. The microfluidic sensor according to any of claims 1 to 15, **characterized in that** it has a boronic acid, a boronic acid derivative or antibodies in an amount of between 0.5 mg and 10 mg as affinity agent in the affinity units (12) and (26).

17. The microfluidic sensor according to any of claims 1 to 14, **characterized in that** it has a quinone, quinone derivative, a quinoid redox dye from the group of phenazines, phenoxazines, phenothiazines, indamines, metallocenes or a redox-active metal complex in an amount of between 0.5 mg and 10 mg as redox mediator in the respective measurement chambers.

18. The microfluidic sensor according to any of claims 1 and 5 to 14, **characterized in that** it has a charged porous support material in the form of a charged polymer or ion exchanger in the flow section (25) as affinity agent for binding non-glycated hemoglobin.

19. A method for indirect detection of glycated hemoglobin, **characterized in that** a microfluidic sensor according to any of claims 1 to 4 and 8 to 17 is placed in an adapted glucose meter, contacted with a sample to be analyzed, a polarization potential is applied to the working electrodes of a potentiostatic three-electrode array, and the amperometrically generated, time-shifted current measurement signals for possible interferences, total content of hemoglobin and content of non-glycated hemoglobin are determined on the basis of the individual sample processing steps during passage of the sample in the respective measurement chambers, and the relative content of glycated hemoglobin is subsequently calculated by forming the difference between the measured signals from the content of total hemoglobin and non-glycated hemoglobin and the ratio of the difference to the total hemoglobin value.

20. A method for direct detection of glycated hemoglobin, **characterized in that** a microfluidic sensor according to any of claims 1 and 5 to 18 is placed in an adapted glucose meter, contacted with a sample to be analyzed, a polarization potential is applied to the working electrodes of a potentiostatic three-electrode array, and the amperometrically generated, time-shifted current measurement signals for total content of hemoglobin, content of residual non-glycated hemoglobin still contained in the sample after sample processing, and content of glycated hemoglobin after accumulation thereof in the third measurement chamber 14 are determined on the basis of the individual sample processing steps during passage of the sample from the intake at the capillary gap 6a of the sample-receiving chamber 6 all the way to the outlet channels 15, 16 17 in the respective measurement chambers, and the relative content of glycated hemoglobin is determined by forming the difference between the measured value of glycated hemoglobin determined in the third measurement chamber 14 and the measured value of residual, non-glycated hemoglobin determined in the second measurement chamber 11 and forming the ratio thereof to the total hemoglobin content, the value of which has been determined in the first measurement chamber 7.

## Revendications

1. Capteur microfluidique voltampérométrique destiné à la détection directe ou indirecte d'hémoglobine glyquée, **caractérisé par**
- un matériau de support planaire (1), qui comprend trois agencements à trois électrodes voltampérométriques empreints de manière géométriquement identique et connectés en parallèle, constitués respectivement d'électrodes de travail, de référence et de contre-électrodes,
- une couche d'isolation (1a) se trouvant au-dessus de celui-ci, comportant des fenêtres de mesure au-dessus des agencements respectifs d'électrode et
- un film plastique microstructuré (5), comprenant une chambre de réception d'échantillon (6), trois chambres de mesure (7), (11) et (14), une chambre d'hémolyse (9) destinée à l'hémolyse des érythrocytes et au moins une chambre d'affinité (12) destinée à lier l'hémoglobine glyquée, ainsi que des canaux de transfert et de sortie, les chambres de réaction et de mesure (6), (7), (9), (11), (12), (14) étant dopées avec des réactifs de lyse cellulaire, de séparation, de réaction d'affinité et de réactions chimiques, électrochimiques ou enzymatiques,
et dans lequel le support planaire (1), la couche d'isolation (1a) et le film (5) sont reliés l'un à l'autre par complémentarité de formes et de façon irréversible de telle sorte que la chambre de réception d'échantillon (6) forme une fente (6a) pour la réception de l'échantillon dans le film (5) avec le support (1) et les chambres de mesure sont positionnées respectivement au-dessus des agencements d'électrode sur le matériau de support (1).

2. Capteur microfluidique destiné à la détection directe de l'hémoglobine glyquée selon la revendication 1, **caractérisé en ce que** le film plastique microstructuré (5) comprend :
la chambre de réception d'échantillon (6), qui est reliée à la chambre d'hémolyse (9), la chambre d'hémolyse (9), qui comprend un détergent pour l'hémolyse des érythrocytes, est reliée d'une part à la première chambre de mesure (7) pourvue d'un médiateur redox destiné à déterminer la teneur en hémoglobine totale de l'échantillon, et est reliée, par l'intermédiaire d'un segment d'écoulement (25) dopé de telle sorte que l'hémoglobine non glyquée est liée ou entravée dans son écoulement de manière perceptible, à la chambre d'affinité (12) dopée avec au moins un agent affine destiné à lier l'hémoglobine glyquée, et à laquelle la seconde chambre de mesure (11),
dopée avec un médiateur destiné à déterminer l'hémoglobine non glyquée restante, est raccordée, moyennant quoi un canal (10) bifurquant en aval du segment d'écoulement (25) garantit d'une part la liaison vers la chambre d'affinité (12) et d'autre part est relié par l'intermédiaire d'une section de retardement (26) à la troisième chambre de mesure (14), qui contient également un agent affine destiné à lier l'hémoglobine glyquée et est dopée avec un médiateur destiné à déterminer l'hémoglobine glyquée, ladite troisième chambre de mesure (14) étant suivie par un canal de résidu d'échantillon (27) destiné à concentrer l'hémoglobine glyquée dans la troisième chambre de mesure 14.

3. Capteur microfluidique selon la revendication 1 ou 2, **caractérisé en ce que** les médiateurs redox et les additifs dopés respectivement dans les chambres de mesure sont identiques dans l'espèce et la concentration.

4. Capteur microfluidique selon l'une des revendications 1 à 3, **caractérisé par**
a. un matériau de support (1), qui comprend des agencements de trois électrodes (2), (3) et (4) voltampérométriques, connectés en parallèle, empreints de manière géométriquement identique en trois fois, constitués respectivement d'électrodes de travail, de référence et de contre-électrodes (18, 18a) (19, 19a), (20, 20a) et qui sont reliés par l'intermédiaire de conducteurs aux surfaces de contact (21, 21a, 22, 22a, 23, 23a),
b. une couche de vernis d'isolation (1a),
c. un film plastique (5) microstructuré comprenant
i. la chambre de réception d'échantillon (6) contenant un agent d'antidote, à l'extrémité de laquelle une ouverture circulaire (6b) se trouve, qui conduit dans
ii. un canal de transfert (8), qui est suivi de la chambre d'hémolyse (9) contenant un détergent hémolytique,
iii. un canal (7a) conduisant à l'extérieur de la chambre d'hémolyse (9), et menant à la première chambre de mesure (7) contenant un médiateur,
iv. un segment d'écoulement (25) menant à l'extérieur de la chambre d'hémolyse (9), comportant éventuellement des segments individuels contenant un agent affine,
v. le canal bifurquant (10), qui conduit d'une part à la chambre d'affinité (12) comportant éventuellement des segments individuels contenant un agent affine, suivie par la seconde chambre de mesure (11) contenant un médiateur, et qui mène d'autre part à
vi. la section de retardement (26), suivie de la troisième chambre de mesure (14) contenant un médiateur, comportant un agent affine vis-à-vis de l'hémoglobine glyquée, et comportant un canal de résidu d'échantillon (27),
vii. des canaux de sortie (15, 16, 17) connectés respectivement en aval des chambres de mesure et comportant des ouvertures de sortie (15a, 15b, 15c).

5. Capteur microfluidique de détection indirecte de l'hémoglobine glyquée selon la revendication 1, **caractérisé en ce que** le film plastique microstructuré (5) comprend :
la chambre de réception d'échantillon (6) comportant au moins un agent d'antidote pour la réduction de la méthémoglobine, qui est suivie de la chambre d'hémolyse (9) comportant au moins un détergent pour l'hémolyse des érythrocytes, qui est elle-même reliée, d'une part, à la chambre de mesure (11) dopée avec au moins un médiateur redox pour déterminer l'hémoglobine totale et, d'autre part, à la chambre d'affinité (12), qui est dopée avec au moins un agent affine pour lier l'hémoglobine glyquée, qui est suivie de la chambre de mesure additionnelle (14), également dopée avec un médiateur, destinée à déterminer l'hémoglobine non glyquée et
**en ce que** la chambre de mesure (7), qui se trouve parallèlement à la chambre d'hémolyse (9) de manière attenante à la chambre de réception d'échantillon (6), est dopée avec au moins un médiateur redox pour déterminer les interférences provoquées par la présence de substances électrochimiques.

6. Capteur microfluidique selon la revendication 5, **caractérisé en ce que** les médiateurs redox et les additifs dopés respectivement dans les chambres de mesure sont identiques en espèce et concentration.

7. Capteur microfluidique selon l'une des revendications 1, 5 ou 6, **caractérisé par**
a. un matériau de support (1), qui comprend des agencements de trois électrodes (2, 3 et 4) voltampérométriques, connectés en parallèle, en trois fois, empreints de manière géométriquement identique, constitués respectivement d'électrodes de travail, de référence et de contre-électrodes (18, 18a) (19, 19a), (20, 20a) et qui sont reliés par l'intermédiaire de conducteurs aux surfaces de contact (21, 21a, 22, 22a, 23, 23a),
b. une couche de vernis d'isolation (1a),
c. un film plastique (5) microstructuré comportant
i. une chambre de réception d'échantillon (6) contenant un agent d'antidote,
ii. la première chambre de mesure (7) contenant un médiateur,
iii. un canal de transfert (8) parallèle à celle-ci, suivi de la chambre d'hémolyse (9) contenant un détergent hémolytique,
iv. un canal bifurquant (10) conduisant à l'extérieur de la chambre d'hémolyse (9), et via
v. un canal direct (13) à la seconde chambre de mesure (11) contenant un médiateur et qui est parallèle à
vi. la chambre d'affinité (12), comportant éventuellement des segments individuels (12a) contenant un agent affine, suivie par une troisième chambre de mesure (14) contenant un médiateur, et
vii. des canaux de sortie (15, 16, 17) connectés respectivement en aval des chambres de mesure et comportant des ouvertures de sortie (15a, 15b, 15c).

8. Capteur microfluidique selon l'une quelconque des revendications 1 à 7, **caractérisé en ce que** le film plastique (5) a une épaisseur de 100 µm à 500 µm.

9. Capteur microfluidique selon l'une quelconque des revendications 1 à 8, **caractérisé en ce que** les microstructures dans le film plastique (8) ont sous forme de chambres et de canaux des profondeurs entre 20 µm et 100 µm et des largeurs ou des longueurs entre 20 µm et 4000 µm.

10. Capteur microfluidique selon l'une quelconque des revendications 1 à 9, **caractérisé en ce que** le volume de la chambre de réception d'échantillon (6) est compris entre 1 µl et 10 µl, de préférence entre 2 µl et 5 µl et le volume total de toutes les autres chambres et des canaux de jonction n'est pas supérieur à celui de la chambre de réception d'échantillon (6).

11. Capteur microfluidique selon l'une quelconque des revendications 1 à 10, **caractérisé en ce qu'**il comprend, entre la couche d'isolation (1a) et le film plastique microstructuré (5), un film adhésif (1b) comportant des fenêtres de mesure au-dessus des agencements d'électrodes respectifs et un évidemment 6c au-dessus de la chambre de réception d'échantillon 6.

12. Capteur microfluidique selon la revendication 11, **caractérisé en ce que** le film adhésif (1b) a une épaisseur de 30 µm à 50 µm et est assemblé aux deux côtés avec le support planaire (1) et le film plastique (5) presque par complémentarité de formes.

13. Capteur microfluidique selon l'une quelconque des revendications 1 à 12, **caractérisé en ce que** le film plastique (5) est constitué de polycarbonate ou de polyester.

14. Capteur microfluidique selon l'une quelconque des revendications 1 à 13, **caractérisé en ce qu'**il comprend, en tant qu'antidote dans la chambre de réception d'échantillon (6), un colorant redox quinoïde, de préférence du bleu de toluidine ou du bleu de méthylène en une quantité entre 0,1 mg et 1 mg.

15. Capteur microfluidique selon l'une quelconque des revendications 1 à 14, **caractérisé en ce qu'**il comprend dans la chambre d'hémolyse (9), en tant que détergent hémolytiquement actif, de la saponine ou un tensioactif non ionique, de préférence de la saponine en une quantité entre 0,05 mg et 1 mg.

16. Capteur microfluidique selon l'une quelconque des revendications 1 à 15, **caractérisé en ce qu'**il comprend, en tant qu'agent affine dans les agencements d'affinité (12 et 26), de l'acide boronique, un dérivé d'acide boronique ou un anticorps en une quantité entre 0,5 mg et 10 mg.

17. Capteur microfluidique selon l'une quelconque des revendications 1 à 14, **caractérisé en ce qu'**il comprend, en tant que médiateur redox dans les chambres de mesure respectives, une quinone, un dérivé de quinone, un colorant redox quinoïde issu du groupe comprenant la phénazine, la phénoxazine, la phénothiazine, l'indamine, les métallocènes ou un complexe métallique à activité redox en une quantité comprise entre 0,5 mg et 10 mg.

18. Capteur microfluidique selon l'une quelconque des revendications 1 et 5 à 14, **caractérisé en ce qu'**il comprend, dans la trajectoire d'écoulement (25), en tant qu'agent affine de liaison de l'hémoglobine non glyquée, une matière de support poreuse chargée sous forme d'un polymère ou d'un échangeur d'ions chargé.

19. Méthode de détection indirecte d'hémoglobine glyquée **caractérisé en ce qu'**on dispose un capteur microfluidique selon l'une des revendications 1 à 4 et 8 à 17 dans un glucomètre adapté, on le met en contact avec un échantillon à analyser, on applique un potentiel de polarisation aux électrodes de travail d'un agencement à trois électrodes potentiostatique, on détermine les signaux de courant générés ampérométriquement, indiquant d'éventuelles interférences, la teneur totale en hémoglobine et la teneur en hémoglobine non glyquée, sur la base des différentes étapes de traitement d'échantillon lors du passage de l'échantillon dans les chambres de mesure respectives et on calcule ensuite la teneur relative en hémoglobine glyquée en effectuant la différence entre les signaux de mesure de la teneur en hémoglobine totale et ceux de la teneur en hémoglobine non glyquée et en effectuant le rapport de la différence obtenue à la valeur de l'hémoglobine totale.

20. Méthode de détection directe d'hémoglobine glyquée **caractérisé en ce qu'**on dispose un capteur microfluidique selon l'une des revendications 1 et 5 à 18 dans un glucomètre adapté, on le met en contact avec un échantillon à analyser, on applique un potentiel de polarisation aux électrodes de travail d'un agencement à trois électrodes potentiostatique, on détermine les signaux de courant générés ampérométriquement, différés dans le temps, indiquant la teneur totale en hémoglobine, la teneur en hémoglobine non glyquée résiduelle contenue encore dans l'échantillon après le traitement et la teneur en hémoglobine glyquée après sa concentration dans la troisième chambre de mesure 14, sur la base des différentes étapes de traitement d'échantillon du passage de l'échantillon de la réception dans la fente capillaire 6a jusqu'aux canaux de sortie 15, 16, 17 dans les chambres de mesure respectives, moyennant quoi, pour déterminer la proportion relative d'hémoglobine glyquée, on effectue la différence entre la valeur d'hémoglobine glyquée déterminée dans la troisième chambre de mesure 14 et la valeur d'hémoglobine non glyquée, résiduelle déterminée dans la seconde chambre de mesure 11 et on calcule son rapport à la teneur en hémoglobine totale, dont la valeur a été déterminée dans la première chambre de mesure 7.
